# EUROPEAN PATENT APPLICATION

(11) **EP 4 335 444 A2**
(43) Date of publication of application: **13.03.2024**
(21) Application number: 23211804.2
(22) Date of filing: 17.01.2018
(51) Int. Cl.: A61K 35/12

(54) **ENGINEERED CELLS FOR INDUCING TOLERANCE**

(30) Priority: 19.01.2017 WO PCT/EP2017/051068
(62) Divisional of application: 18701425.3
(71) Applicant: BioNTech SE, 55131 Mainz (DE); University Of Verona, 37134 Verona (IT)
(72) Inventor: SAHIN, Ugur, 55116 Mainz (DE); BRONTE, Vincenzo, 35031 Abano Terme (PD) (IT); UGEL, Stefano, 31024 Ormelle (TV) (IT); FIORE, Alessandra, 10137 Torino (TO) (IT)
(74) Representative: Schnappauf, Georg

(57) **Abstract**

The present invention relates to cells which are engineered so as to increase the cellular level of cellular FLICE [Fas-associated death domain (FADD)-like IL-1β-converting enzyme]-inhibitory protein (CFLAR). The engineered cells have the ability to induce tolerance. Enhanced tolerogenicity is useful for prolonging survival of a foreign transplant and for treatment of autoimmune diseases.

## Description

### TECHNICAL FIELD OF THE INVENTION

The present invention relates to cells which are engineered so as to increase the cellular level of cellular FLICE [Fas-associated death domain (FADD)-like IL-1β-converting enzyme]-inhibitory protein (CFLAR). The engineered cells have the ability to induce tolerance. Enhanced tolerogenicity is useful for prolonging survival of a foreign transplant and for treatment of autoimmune diseases.

### BACKGROUND OF THE INVENTION

The evolution of the immune system resulted in vertebrates in a highly effective network based on two types of defense: the innate and the adaptive immunity. In contrast to the evolutionary ancient innate immune system that relies on invariant receptors recognizing common molecular patterns associated with pathogens, the adoptive immunity is based on highly specific antigen receptors on B cells (B lymphocytes) and T cells (T lymphocytes) and clonal selection. While B cells raise humoral immune responses by secretion of antibodies, T cells mediate cellular immune responses leading to destruction of recognized cells and play a central role in cell-mediated immunity in humans and animals.

Mature T cells recognize and respond to antigen through their antigen-specific receptors (TCR) which interact with immunogenic peptides (epitopes) bound to major histocompatibility complex (MHC) molecules and presented on the surface of target cells. For example, cytotoxic T cells respond to an antigen that is presented in association with MHC-I proteins. Helper T cells recognize antigen presented on MHC-II proteins. The most immediate consequence of TCR activation is the initiation of signaling pathways resulting in clonal expansion of T cells, upregulation of activation markers on the cell surface, differentiation into effector cells, induction of cytotoxicity or cytokine secretion and induction of apoptosis. The TCR is a part of a complex signaling machinery, which includes the heterodimeric complex of the TCR α- and β-chains, the co-receptor CD4 or CD8 and the CD3 signal transduction modul. While the CD3 chains transfer the activation signal inside the cell, the TCR α/β heterodimer is solely responsible for antigen recognition.

In addition to the critical roles that T cells play in the immune response, dendritic cells (DCs) are equally important. DCs are professional antigen-presenting cells having a key regulatory role in the maintenance of tolerance to self-antigens and in the activation of innate and adaptive immunity against foreign antigens.

By producing soluble factors such as cytokines, interleukins and metabolites, tumours induce an alternative haematopoiesis that modifies the normal myeloid cell differentiation, pushing the proliferation and expansion of cells with immunosuppressive functions called myeloid-derived suppressor cells (MDSCs) (Ugel S et al., 2015, J Clin Invest. 125:3365-76; Marvel D et al., 2015, J Clin Invest. 125:3356-64). The hallmarks of MDSCs are the myeloid origin, the heterogeneous cell composition, and the ability to regulate negatively the adaptive and innate immune response. MDSC functional activity is related to the up-regulation of immune suppressive enzymes, such as arginase 1 (ARG1), indoleamine 2,3-dioxygenase 1 (IDO1), NADPH oxidase (NOX) and inducible nitric oxide synthase (iNOS, also referred as NOS2), as well as an increased production of nitric oxide (NO) and reactive oxygen and nitrogen species (ROS and RNS), such as the free radical peroxynitrite (ONOO⁻) (Bronte V et al., 2005, Nat Rev Immunol. 5:641-54) that inhibit T cell vitality and fitness. MDSC composition is flexible and peculiar for each cancer type and it often changes, following the kinetics and development of the pathology: activated MDSCs are mostly present in the primary tumour and in the metastatic sites but can be also found in circulation, suggesting that MDSC specific activation markers could be used to discriminate them from the other non-suppressive myeloid cells (Gabrilovich DI et al., 2012, Nat Rev Immunol. 12:253-68; De Sanctis F et al, 2016, Biochim Biophys Acta. 1865:35-48). To date, human MDSCs have been classified in three main subsets: monocytic (MO)-MDSCs, polymorphonuclear /granulocytic (PMN)-MDSCs and immature (i)-MDSCs. However, differences in the sample preparation procedures and in the analysis of cytofluorimetric data, together with the lack of exclusive MDSC markers have generated a lot of discrepancies between studies, slowing down the MDSC-enumeration in the clinical routine. In the last twenty years, on the contrary, the identification of MDSC-related molecular and biological pathways and the partial clarification of the cell networks in the tumour microenvironment, through the use of innovative tumour mouse models, have generated an increased interest in MDSCs and their function in cancer pathobiology. Recently, the mouse MDSC heterogeneity between the two main subsets has been reported to occur from a different activation of molecular pathways regulating the death-inducing signalling complex (DISC). Monocytic (MO)-MDSCs generation constitutively requires the presence of the anti-apoptotic molecule cellular FLICE [Fas-associated death domain (FADD)-like IL-1β-converting enzyme]-inhibitory protein (CFLAR, also known as c-FLIP)] that controls the extrinsic apoptotic pathway and caspase 8 activation. On the other hand, polymorphonuclear (PMN)-MDSC expansion depends on the MCL-1-mediated control of the intrinsic mitochondrial death pathway (Haverkamp JM et al, 2014, Immunity 41:947-59). CFLAR has 13 different spliced variants, three of which are conveyed as proteins: the 26 kDa short form (c-FLIPs), the 24 kDa Raji form (c-FLIP_{R}, specifically expressed in only some cell lines and human primary T cells) and the 55 kDa long form (c-FLIP_{L}). All the three CFLAR variants contain two death effector domains (DED) that interact with the apoptotic adaptor protein FAAD. The gene encoding CFLAR is evolutionarily conserved in vertebrates and the structure of c-FLIPS is similar to viral FLICE-inhibitory protein (v-FLIP) that is component of viruses of the gamma herpesvirus class, such as the human herpesvirus 8, a Kaposi's sarcoma-associated virus (Safa AR, 2012, Exp Oncol. 34:176-84; Safa AR et al., 2008, Curr Cancer Drug Targets 8:37-46).

The immune system can also produce undesirable effects. For example, autoimmune disorders are characterized by the loss of tolerance against self-antigens, activation of lymphocytes reactive against "self" antigens (autoantigens), and pathological damage in target organs. Furthermore, the immune system can cause rejection of cell, tissue and organ transplants from unrelated donors. The immune system does not distinguish beneficial intruders, such as a transplanted tissue, from those that are harmful, and thus the immune system rejects transplanted tissues or organs. Rejection of transplanted organs is generally mediated by alloreactive T cells present in the host which recognize donor alloantigens or xenoantigens. On the other side, graft versus host disease can occur when allogeneic, immunologically active cells are introduced, transplanted or grafted in a host resulting in a severe, even fatal, reaction due to immunoincompatibility between the host and graft wherein immunocompetent cells of the graft immunologically attack the host.

Therefore, there is a need for agents having tolerogenic or immunsuppressive actions. In particular, there is a need for agents enhancing tolerogenicity in a host.

### DESCRIPTION OF INVENTION

### Summary of the invention

The present invention encompasses compositions, methods and uses for inducing immunosuppression and tolerance as defined by suppression of an immune response to an antigen.

The present invention is based on the concept that increasing the cellular level of cellular FLICE [Fas-associated death domain (FADD)-like IL-1β-converting enzyme]-inhibitory protein (CFLAR) in cells, in particular myeloid cells or progenitor cells thereof, increases the immunosuppressive activity of the cells. The cells in which the cellular level of CFLAR is increased may be present *in vitro,* i.e. the cells may be isolated cells, or *in vivo,* i.e. the cells may be present within a subject or individual. Thus, the present invention provides a method of enhancing the tolerogenic or immunosuppressive potential of cells, in particular myeloid cells or progenitor cells thereof.

The tolerogenic or immunosuppressive cells described herein are useful for prolonging foreign graft survival in a mammalian host and for ameliorating inflammatory-related diseases, such as autoimmune diseases. The tolerogenic or immunosuppressive cells are also useful for suppressing an immune response in the context of gene therapy of a desired gene or exogenous protein-based therapy. Accordingly, the present invention encompasses methods and compositions for reducing and/or eliminating an immune response to a transplant in a recipient by treating the recipient with an amount of tolerogenic or immunosuppressive cells described herein to reduce or inhibit host rejection of the transplant. Transplant refers to any material that is to be administered to a host. For example, a transplant includes, but is not limited a biocompatible lattice, a tissue, an organ, a cell, a nucleic acid, and a polypeptide. Also encompassed are methods and compositions for reducing and/or eliminating an immune response in a host by the foreign transplant against the host, i.e., graft versus host disease, by treating the donor transplant and/or recipient of the transplant with tolerogenic or immunosuppressive cells described herein in order to inhibit or reduce an adverse response by the donor transplant against the recipient. In addition, the present invention encompasses methods and compositions for reducing and/or eliminating an immune response to an exogenously delivered protein in a recipient by treating the recipient with an amount of the tolerogenic or immunosuppressive cells described herein to reduce or inhibit rejection of the protein.

Various aspects of the present invention envision the genetic modification of cells, either *in vitro* or *in vivo,* to express CFLAR or to express increased levels of CFLAR. Different vector formats can be used for genetic modification of cells, such as RNA, DNA or viral vectors that can be applied either *in vitro* or *in vivo* . If the cells in which the cellular level of CFLAR is increased are present *in vitro,* strategies may rely on genetically modifying the cells *in vitro* and transferring the cells into a recipient following an optional expansion of the cells *in vitro.* The cells used for treatment may be autologous, allogeneic or syngeneic to a recipient. If the cells in which the cellular level of CFLAR is increased are present *in vivo,* engineering such as by transfection with nucleic acid of the cells may take place *in situ* in the patient and strategies may rely on genetically modifying the cells *in vivo* by administering an appropriate nucleic acid to a recipient, optionally in an appropriate carrier.

The strategies for inducing immunosuppression and/or tolerance described herein can be used to suppress anticipated, unwanted immune responses, e.g., to prolong gene therapy or recurrent administration of therapeutic proteins, e.g., expressed in a mammalian host by way of a vector or applied exogenously to a mammalian host, to prolong foreign graft survival in a host, to treat or prevent graft versus host disease, to treat or prevent autoimmune diseases, including, but not limited to, autoimmune arthritis, autoimmune diabetes, asthma, septic shock, lung fibrosis, glomerulonephritis, and artherosclerosis.

In one aspect, the invention relates to a population of cells comprising myeloid cells or progenitor cells thereof which are engineered so as to increase the cellular level of cellular FLICE [Fas-associated death domain (FADD)-like IL-1β-converting enzyme]-inhibitory protein (CFLAR). For example, the cellular level of CFLAR may be increased by increasing expression of CFLAR in cells.

In one embodiment, increasing the cellular level of CFLAR increases the immunosuppressive activity of the cells.

In one embodiment, the myeloid cells or progenitor cells thereof are engineered so as to increase cellular expression of CFLAR.

In one embodiment, the myeloid cells or progenitor cells thereof are transfected with nucleic acid encoding CFLAR. In one embodiment, expression of CFLAR is increased in the transfected cells compared to the non-transfected cells. In one embodiment, transfection is a stable or transient transfection. In one embodiment, the myeloid cells or progenitor cells thereof are transfected using viral vectors as carrier such as lentiviral vectors. In one embodiment, said nucleic acid is RNA.

In one embodiment, the CFLAR is CFLAR_{L} and/or CFLAR_{S}.

In one embodiment, the myeloid cells or progenitor cells thereof are isolated and/or purified cells, in particular isolated and/or purified by magnetic cell sorting.

In one embodiment, the myeloid cells or progenitor cells thereof are monocytes. In one embodiment, the myeloid cells or progenitor cells thereof are CD14+ monocytes. In one embodiment, the myeloid cells or progenitor cells thereof are CD14+ monocytes isolated from buffy coats.

In one embodiment, the myeloid cells or progenitor cells thereof are stem cells. In one embodiment, the myeloid cells or progenitor cells thereof are bone marrow CD34+ cells. In one embodiment, the myeloid cells or progenitor cells thereof are bone marrow CD34+ cells isolated from bone marrow mononuclear cells (MNC).

In one embodiment, the cells have been treated so as to promote the acquisition of immunosuppressive activity. In one embodiment, treatment so as to promote the acquisition of immunosuppressive activity comprises *in vitro* differentiation in the presence of G-CSF and GM-CSF.

In one embodiment, the population of cells of the invention also comprises myeloid cells or progenitor cells thereof which are not engineered so as to increase the cellular level of CFLAR. In one embodiment, the population of cells of the invention only comprises myeloid cells or progenitor cells thereof which are engineered so as to increase the cellular level of CFLAR. In one embodiment, the population of cells of the invention comprises cells other than myeloid cells or progenitor cells thereof. In one embodiment, the population of cells of the invention only comprises myeloid cells or progenitor cells thereof.

In a further aspect, the invention relates to the population of cells described herein for therapy. In one embodiment, the therapy requires immunosuppression.

In a further aspect, the invention relates to the population of cells described herein for treating a patient in need of immunosuppression. In one embodiment, the population of cells is autologous to the patient.

In a further aspect, the invention relates to a pharmaceutical composition comprising the population of cells described herein. A pharmaceutical composition of the invention may comprise a pharmaceutically acceptable carrier and may optionally comprise one or more adjuvants, stabilizers etc.

In a further aspect, the invention relates to a pharmaceutical composition described herein for therapy. In one embodiment, the therapy requires immunosuppression.

In a further aspect, the invention relates to a pharmaceutical composition described herein for treating a patient in need of immunosuppression. In one embodiment, the population of cells is autologous to the patient.

In a further aspect, the invention relates to a use of the population of cells described herein for the production of a medicament for therapy. In one embodiment, the therapy requires immunosuppression.

In a further aspect, the invention relates to a use of the population of cells described herein for the production of a medicament for treating a patient in need of immunosuppression. In one embodiment, the population of cells is autologous to the patient.

In a further aspect, the invention relates to a method for treating a patient in need of immunosuppression comprising administering the population of cells described herein. In one embodiment, the population of cells is autologous to the patient.

In a further aspect, the invention relates to a method for treating a patient in need of immunosuppression comprising increasing the cellular level of CFLAR in myeloid cells or progenitor cells thereof of the patient.

In one embodiment, increasing the cellular level of CFLAR increases the immunosuppressive activity of the cells.

In one embodiment, increasing the cellular level of CFLAR in myeloid cells or progenitor cells thereof of the patient comprises administering a nucleic acid encoding CFLAR. In one embodiment, the nucleic acid encoding CFLAR transfects myeloid cells or progenitor cells thereof. In one embodiment, said transfection is a stable or transient transfection. In one embodiment, the myeloid cells or progenitor cells thereof are transfected using viral vectors as carrier such as lentiviral vectors. In one embodiment, said nucleic acid is RNA.

In one embodiment, the CFLAR is CFLAR_{L} and/or CFLAR_{S}.

In one embodiment, the myeloid cells or progenitor cells thereof are monocytes. In one embodiment, the myeloid cells or progenitor cells thereof are CD14+ monocytes.

In one embodiment, the myeloid cells or progenitor cells thereof are stem cells. In one embodiment, the myeloid cells or progenitor cells thereof are bone marrow CD34+ cells.

In a further aspect, the invention relates to a nucleic acid encoding CFLAR for therapy. In one embodiment, the therapy requires immunosuppression.

In a further aspect, the invention relates to a nucleic acid encoding CFLAR for treating a patient in need of immunosuppression.

In a further aspect, the invention relates to a pharmaceutical composition comprising a nucleic acid encoding CFLAR.

In a further aspect, the invention relates to a pharmaceutical composition described herein for therapy. In one embodiment, the therapy requires immunosuppression.

In a further aspect, the invention relates to a pharmaceutical composition described herein for treating a patient in need of immunosuppression.

In a further aspect, the invention relates to a use of a nucleic acid encoding CFLAR for the production of a medicament for therapy. In one embodiment, the therapy requires immunosuppression.

In a further aspect, the invention relates to a use of a nucleic acid encoding CFLAR for the production of a medicament for treating a patient in need of immunosuppression.

In one embodiment of all aspects described herein, a therapy requiring immunosuppression is transplantation and a patient in need of immunosuppression is a transplant recipient. Thus, the invention also encompasses the treatment of a transplant recipient to reduce in the recipient an immune response against the transplant and/or to treat or prevent rejection of the transplant by the recipient. In different embodiments, the transplant is selected from the group consisting of a tissue, an organ, a cell, a nucleic acid, a protein, a biocompatible lattice, and any combination thereof. In some aspects, the treatments described herein for inducing immunosuppression and tolerance are administered to the recipient prior to the transplant. In other aspects, the treatments described herein for inducing immunosuppression and tolerance are administered to the recipient concurrently with the transplant. In yet other aspects, the treatments described herein for inducing immunosuppression and tolerance are administered as part of the transplant. In still another aspect, the treatments described herein for inducing immunosuppression and tolerance are administered to the recipient subsequent to the transplant.

In one embodiment of all aspects described herein, a therapy requiring immunosuppression is the treatment or prevention of graft versus host disease and a patient in need of immunosuppression is a patient having graft versus host disease or being at risk of developing graft versus host disease.

In one embodiment of all aspects described herein, a therapy requiring immunosuppression is the treatment or prevention of an autoimmune disease and a patient in need of immunosuppression is a patient having an autoimmune disease or being at risk of developing an autoimmune disease.

In one aspect, the invention provides the agents and compositions such as cells and pharmaceutical compositions described herein for use in the methods of treatment described herein.

In one aspect, the invention provides a viral vector comprising nucleic acid encoding CFLAR. In one embodiment, the viral vector is a lentiviral vector. The viral vector is preferably useful for engineering or transfecting myeloid cells or progenitor cells thereof *in vivo* or *in vitro.*

Other features and advantages of the instant invention will be apparent from the following detailed description and claims.

### Brief description of the drawings

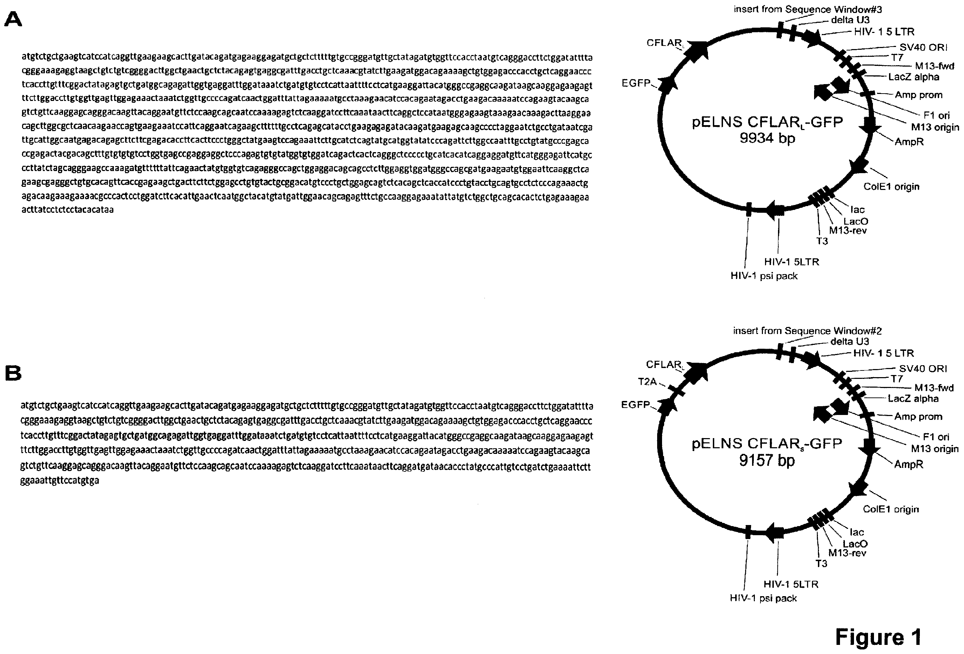

Figure 1 shows the gene sequence of CFLAR long (referred as CFLAR_{L}) with the plasmid used to generate the lentivirus encoding CFLAR_{L} (A) and the gene sequence of CFLAR short (referred as CFLARs) with the plasmid used to generate the lentivirus encoding CFLARs.

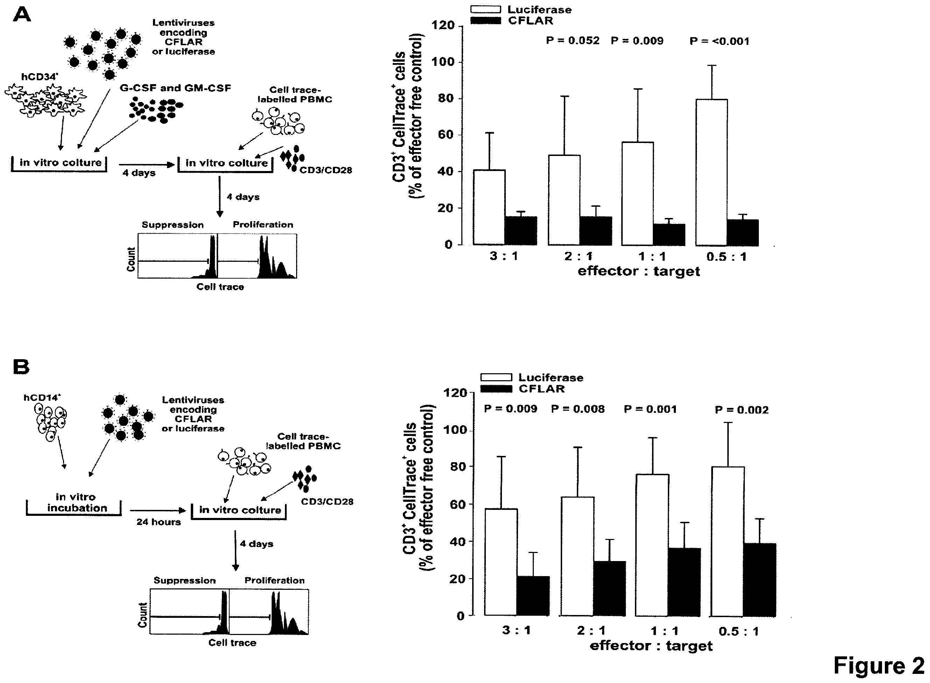

Figure 2 shows the protocol and the results of immunosuppressive assays of infected human CD34⁺ cells expressing CFLAR_{L} and CFLARs to inhibit activated T cells compared to luciferase-expressing CD34⁺ cells used as negative control (A), together with the protocol and the results of immunosuppressive assays of infected human CD14⁺ cells expressing CFLAR_{L} and CFLARs to inhibit activated T cells compared to luciferase-expressing CD14⁺ cells used as negative control (B).

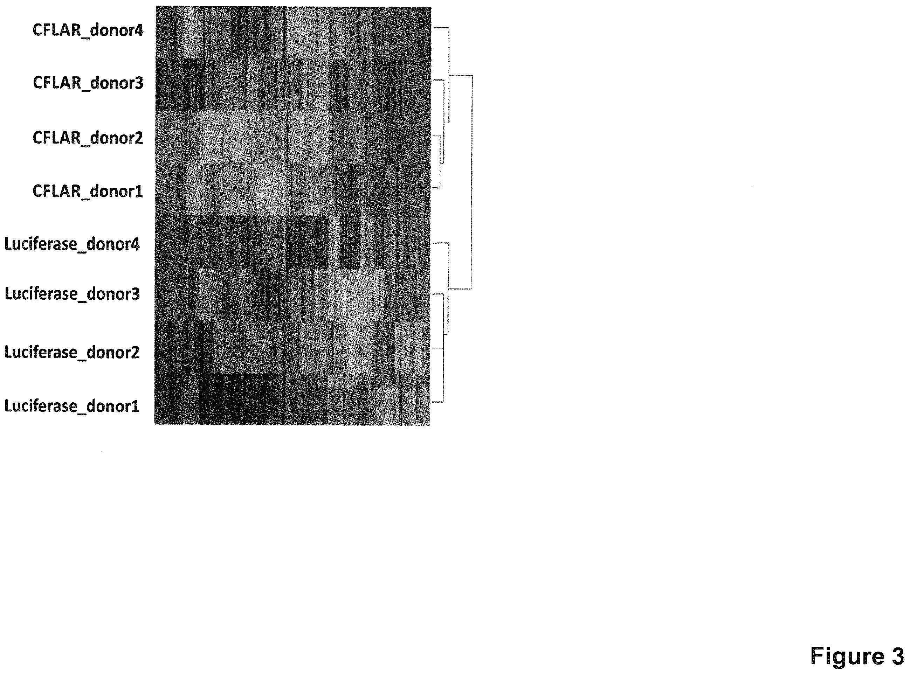

Figure 3 shows the heat map of up-regulated and down-regulated genes mediated by CFLAR-enforced expression on CD14⁺ cells compared to luciferase-infected CD14⁺ cells. The CD14⁺ cells were obtained from four different buffy coats of healthy donors (HD).

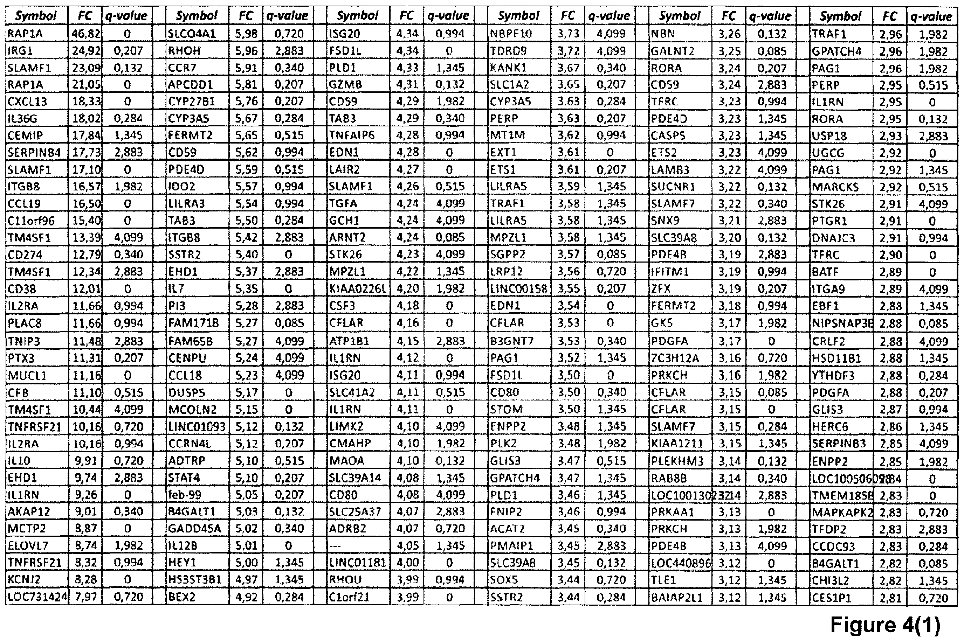

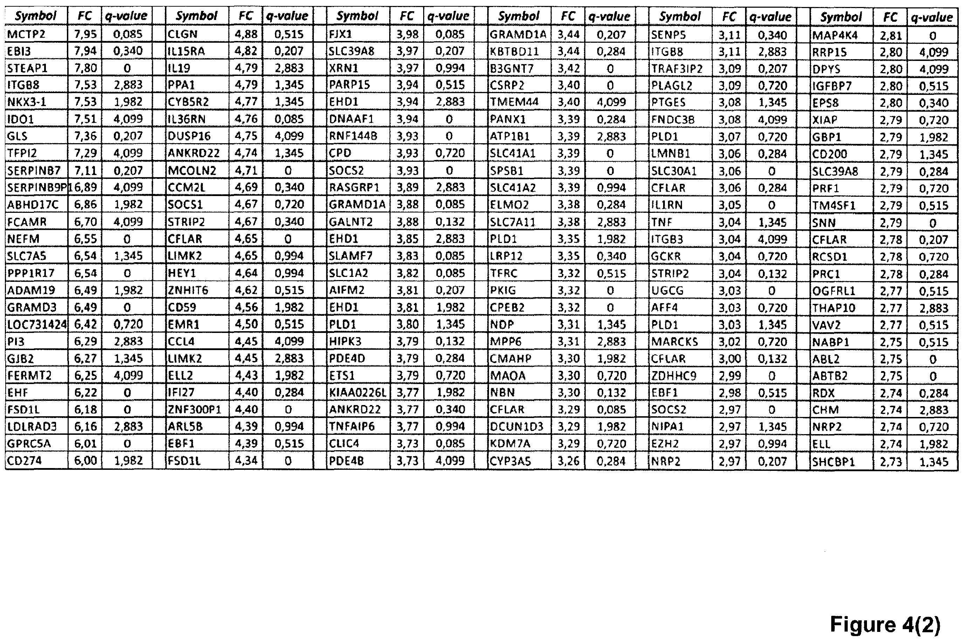

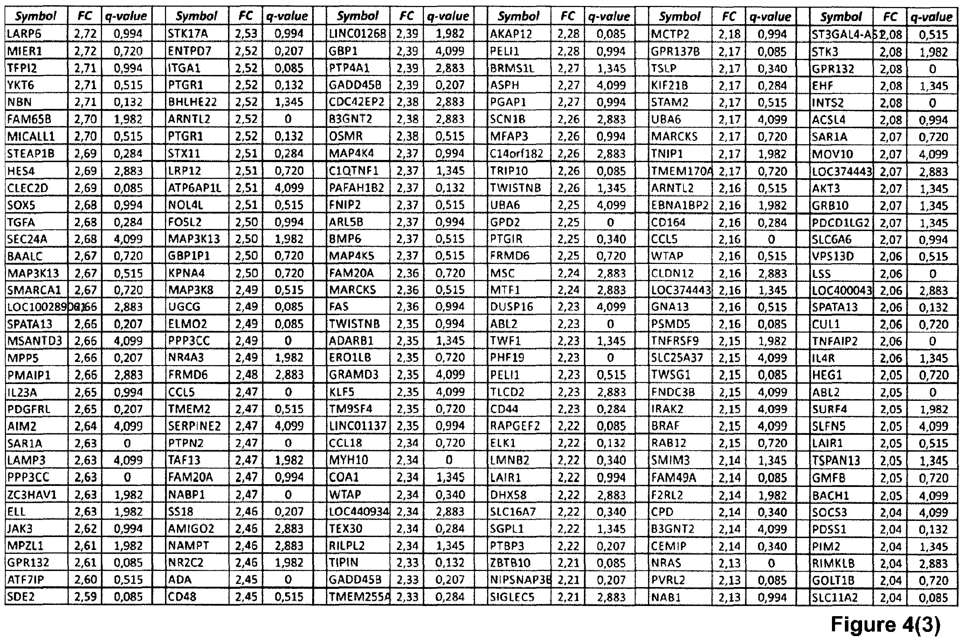

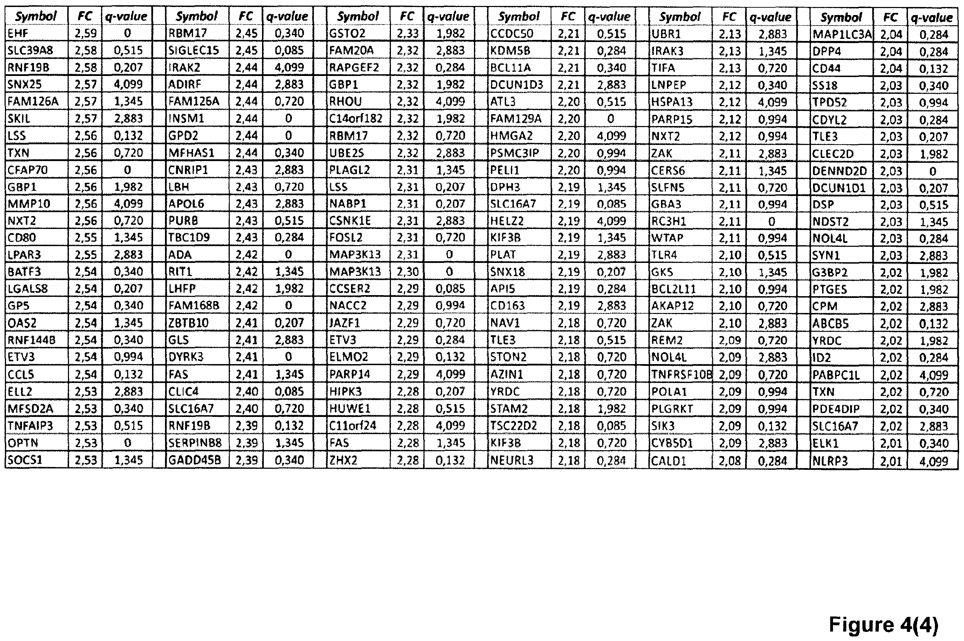

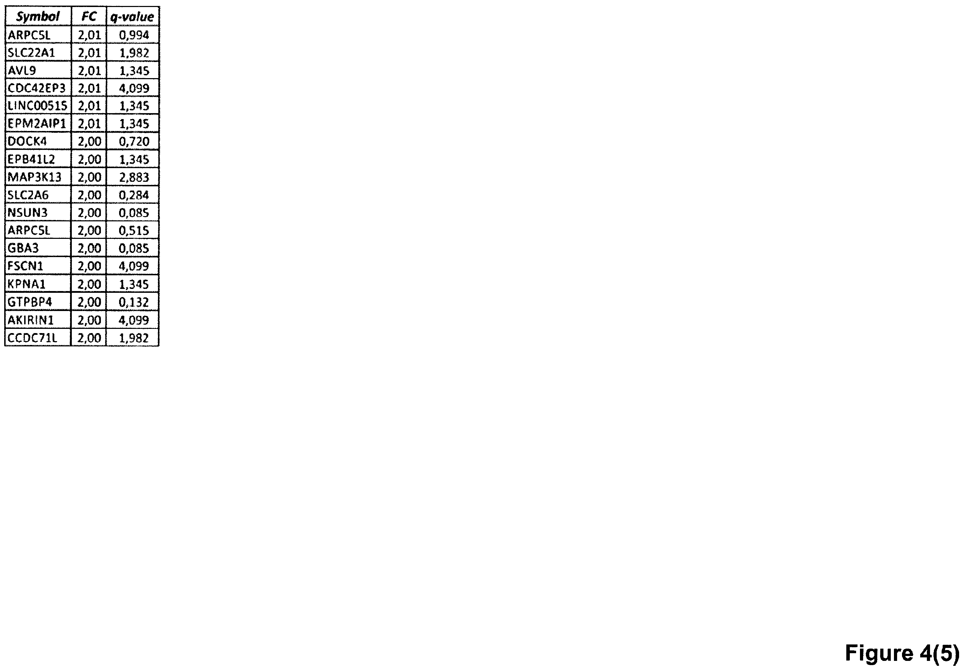

Figure 4 shows the list of significantly up-regulated genes by the induced CFLAR expression by lentivirus on CD14⁺ cells compared to luciferase-infected CD14⁺ cells.

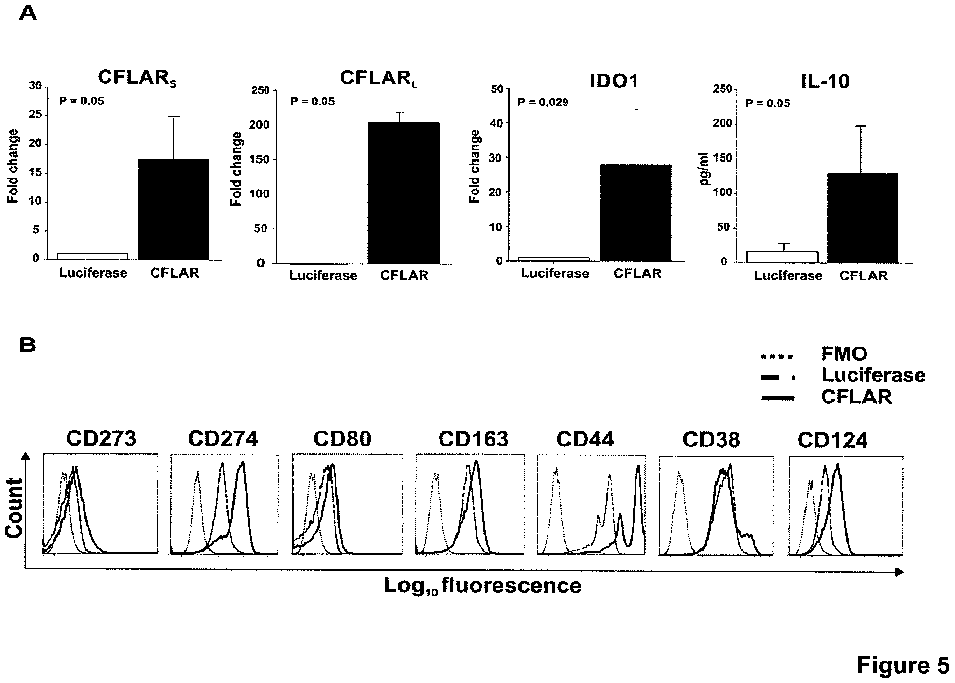

Figure 5 shows validation of some gene targets modulated by the enforced CFLAR expression in CD14⁺ cells: up-regulation of CFLARs, CFLAR_{L} and IDO1 genes by Real Time PCR (A); production of IL-10 by ELISA assay (B); and enhanced expression of surface markers CD273, CD274, CD80, CD163, CD44, CD38 and CD124 by flow cytometry.

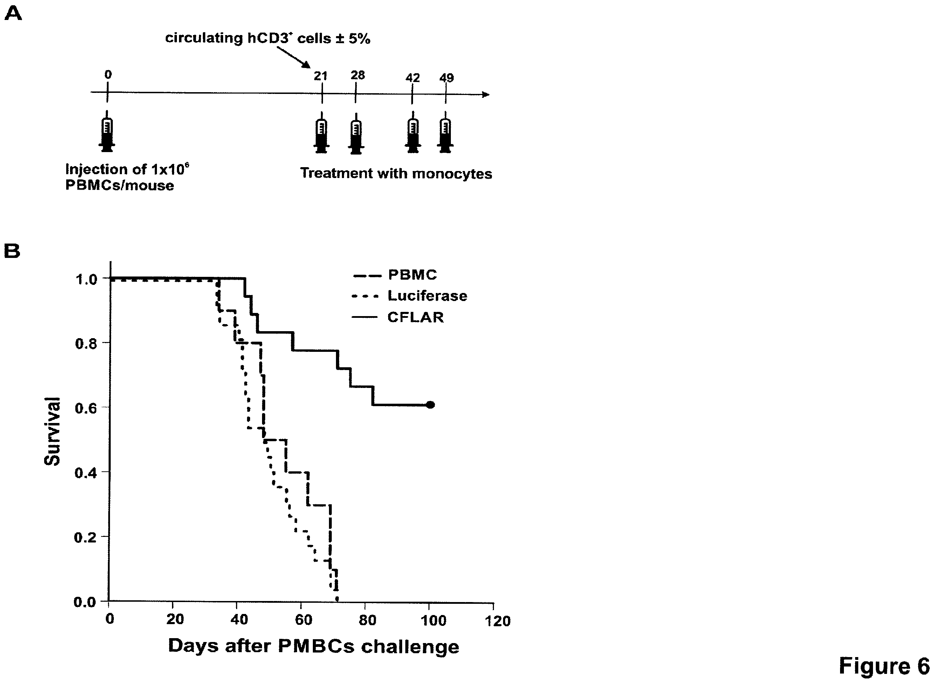

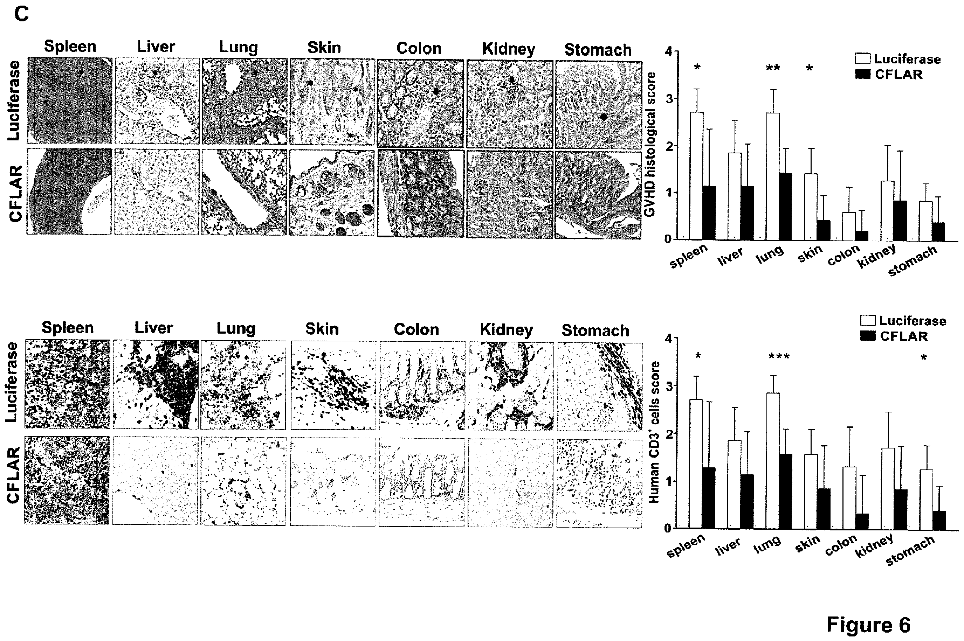

Figure 6 shows the protocol (A) for treating GVHD-undergoing mice engrafted with human PBMCs (the immunomodulating approach was based on the repeated administration of CFLAR-expressing monocytes) and the results of this treatment in prolonging survival of treated mice compared to mice treated with monocytes either un-transduced or transduced with a vector encoding luciferase (used as control); (B), the pathological evaluation by histopatological score (C, upper panels); and the enumeration of tissue-infiltrating human lymphocytes (defined as hCD3⁺ cells) by immunohistochemistry (C, lower panels).

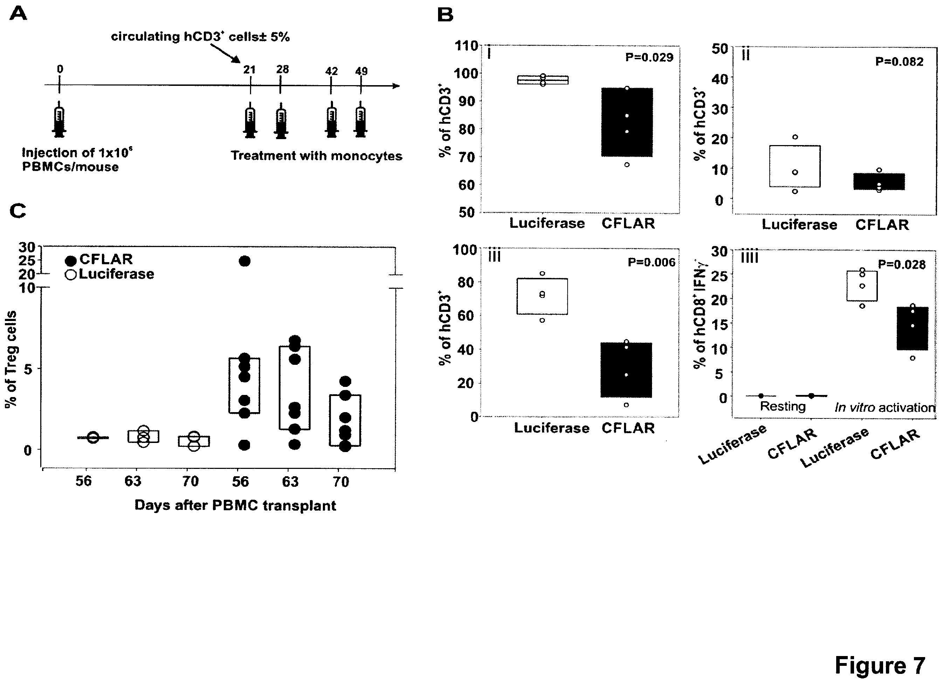

Figure 7 shows the immune evaluation of GVHD-undergoing mice treated with human CD14⁺ cells expressing either CFLAR or luciferase as reported in the protocol scheme (A): mice treated with CFLAR-expressing cells present a lower frequency of circulating hCD3⁺ T cells in the blood (B, panel i), spleen (B, panel ii) and bone marrow (B, iii); moreover GVHD-undergoing mice treated with CFLAR-expressing CD14⁺ cells have a lower frequency of IFN-γ releasing, hCD8⁺ T cells compared to GVHD-undergoing mice treated with luciferase-expressing CD14⁺ after *in vitro* stimulation with PMA (50 ng/ml) plus ionomycin (1 µg/ml); finally, mice treated with repeated injection of CD14⁺ cells expressing CFLAR showed higher amount of regulatory T lymphocytes (Treg) compared to mice treated with CD14⁺ cells expressing luciferase.

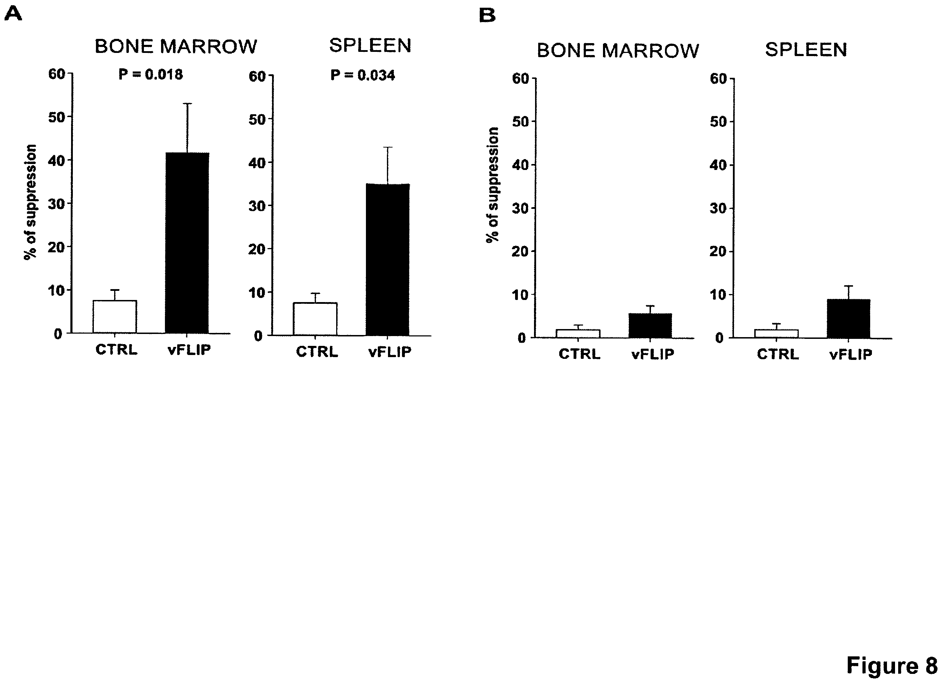

Figure 8 shows the results of the immunosuppressive assay of both bone marrow- and spleen-derived CD11B⁺ Ly6G⁻ Ly6C^{high} monocyte (panel A) and CD11B⁺ Ly6G⁺ Ly6C^{low} granulocytes (panel B) isolated from Rosa26.vFLIP;LysMcre (in black) compared to CD11B⁺ Ly6G⁻ Ly6C^{high} cells isolated from control mice, Rosa26.vFLIP(in white).

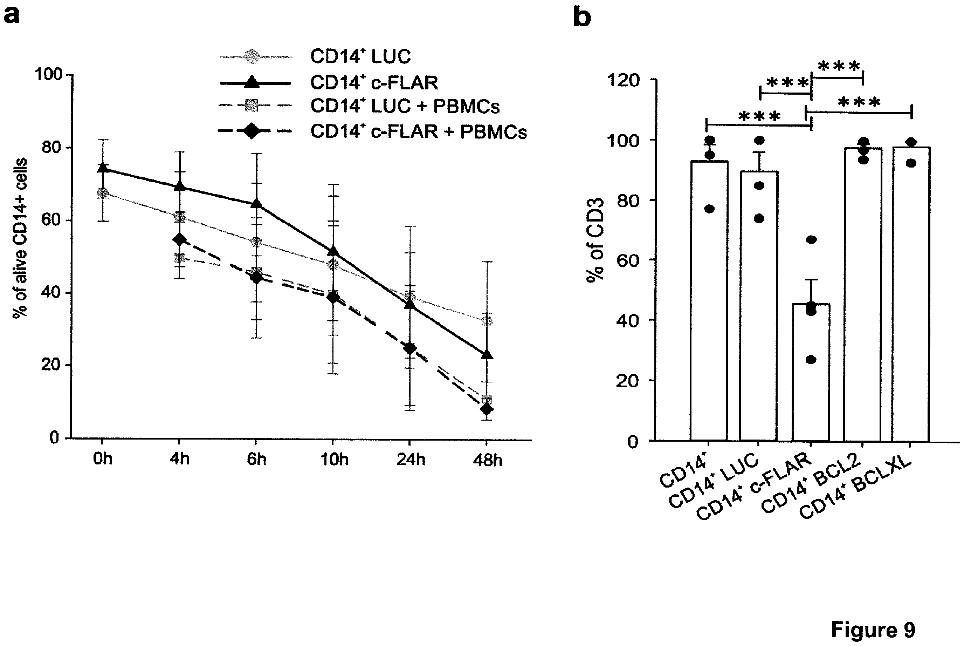

Figure 9: c-FLAR induces an immunosuppressive program independently of its anti-apoptotic functions. a) Evaluation of the *in vitro* vitality of CD14+ cells infected with luciferase (as control) or c-FLAR-expressing lentivirus (monocytes isolated from 4 different buffy coats). b) Only c-FLAR enforced expression induced immunosuppressive functions. In fact monocytes infected with either BCL-2 or BCL-expressing lentivirus did not show immunosuppressive ability. All these data demonstrated that CFLAR drives immunosuppressive functions independently of apoptosis protection.

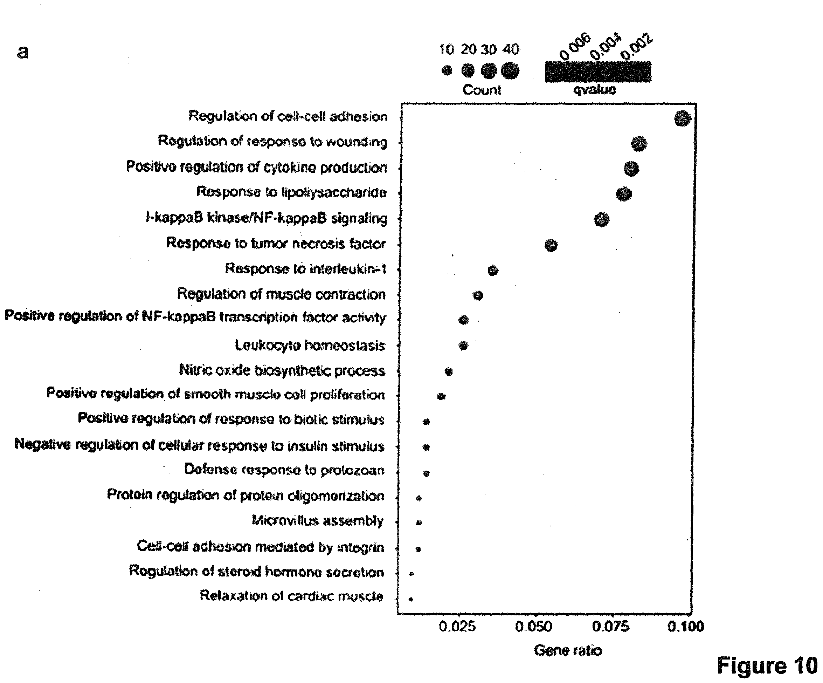

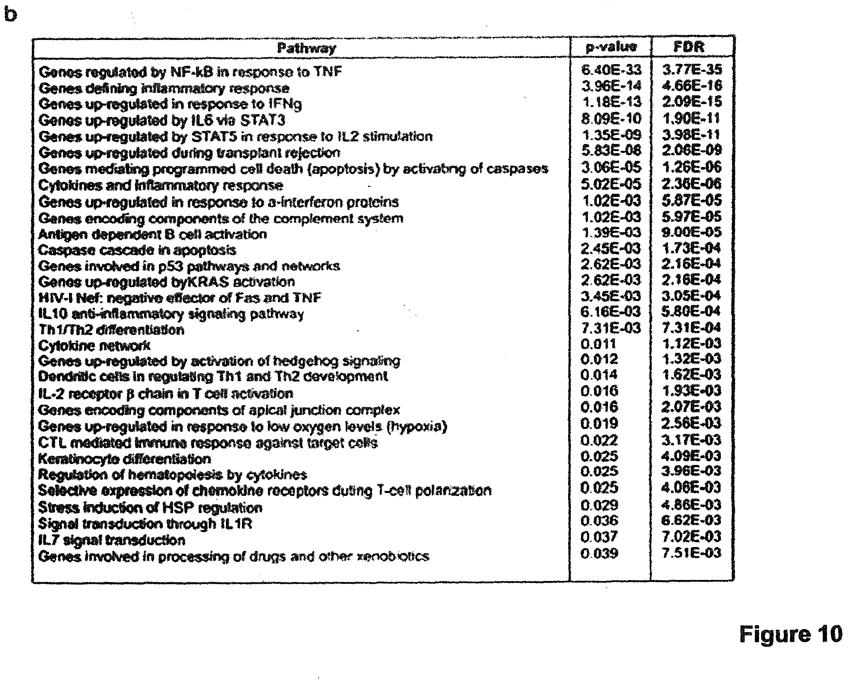

Figure 10: Enforced c-FLAR expression in monocytes drives MDSC-associated molecular programs. a) Enrichment of GO BP terms for the 486 unique up-regulated genes in c-FLAR overexpressed samples. Enrichment has been determined using the GO over-representation test implemented in cluster Profiler and the GO Biological Process categories, p-values have been corrected using BH procedure. b) Enrichment of signaling pathways for up-regulated genes in c-FLAR overexpressed samples (Figures 3, 4a and 4b). Differentially expressed genes have been identified using SAM. Setting q-value<1% resulted in 486 unique genes up-regulated in c-FLAR overexpressed samples (c-FLAR signature). Enrichment has been determined using a Fisher's test on all 267 signaling pathways of GSEA. p-values have been corrected using BH procedure. FDR q-val, false discovery rate q-value.

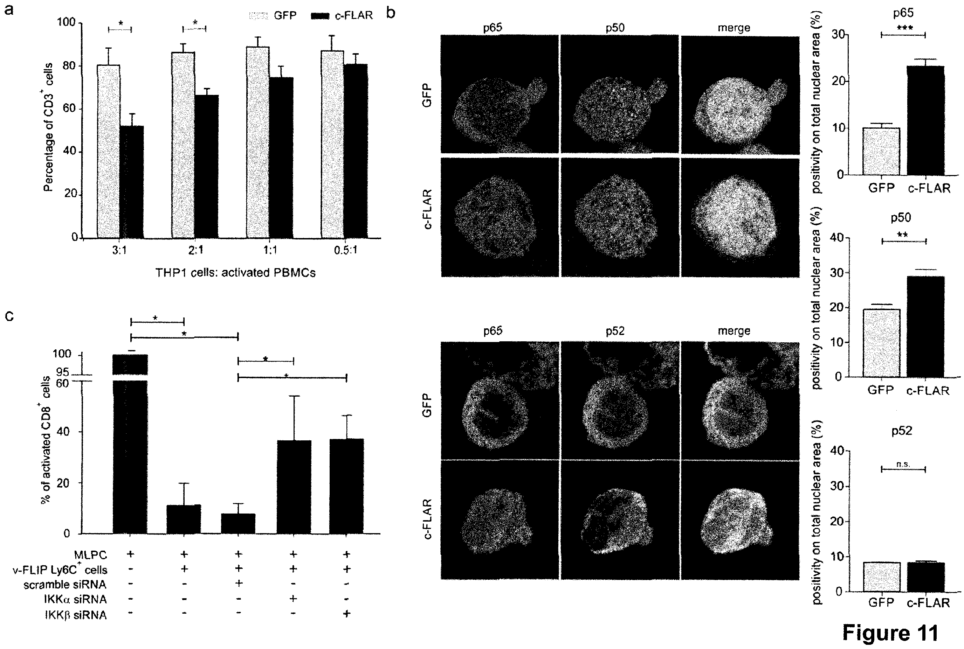

Figure 11: c-FLAR activates the canonical NF-κB pathway in myeloid cells. a) THP1 cells transfected with c-FLAR RNA acquired immunosuppressive functions compared to control (THP1 cells transfected with GFP-encoding RNA). Suppressive activity was measured by enumeration of the absolute number of CD3⁺ T cells collected after co-culture with transfected THP1 cells. Data are presented as mean ± s.e.m of three independent experiments. b) Immunofluorescence confocal microscopy of p65, p50 and p52 nuclear translocation of transfected THP1 cells. Nuclei were counterstained with DAPI and slides were visualized using confocal microscopy. Original images ×800 for all panels, c) v-FLIP expressing Ly6C⁺ cells were purified and transfected with scramble, IKKα and IKKβ siRNA for at least 18h. After that myeloid cells were incubated with CellTrace-labeled OT-I cells and co-cultured in the presence of SIINFEKL peptide for three days. Suppressive activity was measured by enumeration of the absolute number of CD8⁺ T cells collected after co-culture. Data are presented as mean ± s.e.m of four independent experiments.

### Detailed description of the invention

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Preferably, the terms used herein are defined as described in "A multilingual glossary of biotechnological terms: (IUPAC Recommendations)", H.G.W. Leuenberger, B. Nagel, and H. Kölbl, Eds., (1995) Helvetica Chimica Acta, CH-4010 Basel, Switzerland.

The practice of the present invention will employ, unless otherwise indicated, conventional methods of biochemistry, cell biology, immunology, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein.

All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

The term "recombinant" in the context of the present invention means "made through genetic engineering". Preferably, a "recombinant object" such as a recombinant cell or nucleic acid in the context of the present invention is not occurring naturally.

The term "naturally occurring" as used herein refers to the fact that an object can be found in nature. For example, a peptide or nucleic acid that is present in an organism (including viruses) and can be isolated from a source in nature and which has not been intentionally modified by man in the laboratory is naturally occurring.

As used herein, an "isolated" cell is a cell that is essentially free of other cell types. An isolated cell also refers to a cell which has been separated from other cell types with which it is normally associated in its naturally occurring state. In some instances, a population of isolated cells refers to a homogenous population of cells. In other instances, this term refers simply to cells that have been separated from the cells with which they are naturally associated in their natural state. In some embodiments, the cells are cultured *in vitro.* In other embodiments, the cells are not cultured *in vitro.* In one embodiment, a cell population described herein contains 50% or more of the desired cells in the cell population, preferably 75% or more of the cell population, more preferably 85% or 90% or more of the cell population, and most preferably 95% or more of the cell population.

As used herein, a "purified" cell is a cell that is essentially free of undesired components such as cellular material and/or contaminating proteins from the tissue source from which the cell is derived. A purified cell also refers to a cell which has been separated from undesired components.

In one embodiment of the invention, a population of cells comprising myeloid cells or progenitor cells thereof refers to a population of cells that is substantially free of cells other than myeloid cells or progenitor cells thereof and/or undesired components.

As the term is used herein, "separated from" or "separating" refers to the characteristic of a population of first substances being removed from the proximity of a population of second substances, wherein the population of first substances is not necessarily devoid of the second substance, and the population of second substances is not necessarily devoid of the first substance. However, a population of first substances that is "separated from" a population of second substances has a measurably lower content of second substances as compared to the non-separated mixture of first and second substances.

Haematopoietic blood cells are derived from pluripotent bone marrow stem cells by proliferation and differentiation. The stem cells, which are characterised by their repertoire of cell surface receptors (e.g. CD34+), are undifferentiated. Through rounds of self-renewal and expansion by proliferation, followed by differentiation in response to cytokines and growth factors, these cells give rise to all of the mature functional blood cells (monocytes/macrophages, eosinophils, neutrophils, T lymphocytes and B lymphoctes).

In the differentiation of blood cells, two lineage pathways are clearly delineated; myeloid (including monocytes/macrophages, eosinophils and neutrophils) and lymphoid (including T lymphocytes and B lymphoctes). In general terms, myeloid cells perform functional roles associated with the rapid killing and removal of pathogens (such as bacteria and parasites) while the lymphoid cells are associated with the memory of pathogens and the generation of antibody-mediate immune response. Myeloid cells perform a more transient function and so are produced in large numbers, survive days/weeks rather than months or years, and are fully and terminally differentiated, functional specialised cells that do not divide but remain in the GO/GI phase of the cell cycle. Once they have performed their function they die by apoptosis and are removed by phagocytosis. In contrast, and because of their specialised memory role T and B lymphocytes can be long lived and are capable of extensive proliferation and cell cycle.

Accordingly, the term "myeloid cells" designates cells of myeloid origin, encompasses terminally differentiated, non-dividing (i.e. non-proliferative) cells derived from the myeloid cell lineage and includes neutrophils or polymorphonuclear neutrophils (PMNs), eosinophils and mononuclear phagocytes. The latter cells are known as monocytes when in the blood and macrophages when they have migrated into the tissues. Terminal differentiation is the normal endpoint in cellular differentiation and is usually not reversible.

In one particularly preferred embodiment, the term "myeloid cells" relates to monocytes. The term "monocyte" as used herein refers to white blood cells that circulate in the blood stream. Monocytes are found in the circulating peripheral blood where they make up 10-20% of the total mononuclear cell population. Monocytes are characterized by their phenotypic expression of CD14, and thus, are commonly called CD14+ monocytes. They play an important role in host defense as circulating monocytes and can differentiate into tissue macrophages and dendritic cells with potent antigen-presenting capability. Monocytes differentiate into macrophages upon migration into the tissues. Thus, as used in the present invention, the term "monocyte" also refers to macrophages.

The term "buffy coat" relates to the fraction of an anticoagulated blood sample that contains most of the white blood cells and platelets following density gradient centrifugation of the blood.

Progenitor cells of myeloid cells include hematopoietic cells such as hematopoietic stem cells and myeloid progenitor cells. In particular, progenitor cells of myeloid cells include cells expressing CD34+ (CD34+ cells). Cells expressing CD34 are normally found in the umbilical cord and bone marrow as hematopoietic cells. Bone marrow CD34+ cells contain hematopoietic stem and progenitor cells and are known to differentiate into all the various blood cell types. CD34+ cells may be isolated from blood samples using immunomagnetic or immunofluorescent methods.

Terms such as "reducing", "inhibiting" or "decreasing" relate to the ability to cause an overall decrease, preferably of 5% or greater, 10% or greater, 20% or greater, more preferably of 50% or greater, and most preferably of 75% or greater, in the level. These terms include a complete or essentially complete inhibition, i.e. a reduction to zero or essentially to zero.

Terms such as "increasing", "enhancing", or "promoting" relate to the ability to cause an overall increase, preferably of 5% or greater, 10% or greater, 20% or greater, 50% or greater, 75% or greater, 100% or greater, 200% or greater, or 500% or greater, in the level. These terms may relate to an increase, enhancement, or promotion from zero or a non-measurable or non-detectable level to a level of more than zero or a level which is measurable or detectable. Alternatively, these terms may also mean that there was a certain level before an increase, enhancement, or promotion and after the increase, enhancement, or promotion the level is higher.

According to the present invention, the term "increase the cellular level" with respect to a particular substance such as CFLAR relates to measures that result in a higher degree or amount of the substance compared to the normal situation, in particular the normal situation in a cell, wherein the cellular level is not increased/has not been increased by man.

The term "immune response" refers to an integrated bodily response to an antigen and preferably refers to a cellular immune response, a humoral immune response, or both. According to the invention, the term "immune response to" or "immune response against" with respect to an agent such as an antigen, cell or tissue, relates to an immune response such as a cellular response directed against the agent.

The terms "cellular immune response", "cellular response", "cell-mediated immunity" or similar terms are meant to include a cellular response directed to cells characterized by expression of an antigen and/or presentation of an antigen with class I or class II MHC. The cellular response relates to cells called T cells or T lymphocytes which act as either "helpers" or "killers". The helper T cells (also termed CD4⁺ T cells) play a central role by regulating the immune response and the killer cells (also termed cytotoxic T cells, cytolytic T cells, CD8⁺ T cells or CTLs) kill cells such as diseased cells.

The term "humoral immune response" refers to a process in living organisms wherein antibodies are produced in response to agents and organisms, which they ultimately neutralize and/or eliminate. The specificity of the antibody response is mediated by T and/or B cells through membrane-associated receptors that bind antigen of a single specificity. Following binding of an appropriate antigen and receipt of various other activating signals, B lymphocytes divide, which produces memory B cells as well as antibody secreting plasma cell clones, each producing antibodies that recognize the identical antigenic epitope as was recognized by its antigen receptor. Memory B lymphocytes remain dormant until they are subsequently activated by their specific antigen. These lymphocytes provide the cellular basis of memory and the resulting escalation in antibody response when re-exposed to a specific antigen.

The term "antibody" as used herein, refers to an immunoglobulin molecule, which is able to specifically bind to an epitope on an antigen. In particular, the term "antibody" refers to a glycoprotein comprising at least two heavy (H) chains and two light (L) chains interconnected by disulfide bonds. The term "antibody" includes monoclonal antibodies, recombinant antibodies, human antibodies, humanized antibodies, chimeric antibodies and combinations of any of the foregoing. Each heavy chain is comprised of a heavy chain variable region (VH) and a heavy chain constant region (CH). Each light chain is comprised of a light chain variable region (VL) and a light chain constant region (CL). The variable regions and constant regions are also referred to herein as variable domains and constant domains, respectively. The VH and VL regions can be further subdivided into regions of hypervariability, termed complementarity determining regions (CDRs), interspersed with regions that are more conserved, termed framework regions (FRs). Each VH and VL is composed of three CDRs and four FRs, arranged from amino-terminus to carboxy-terminus in the following order: FR1, CDR1, FR2, CDR2, FR3, CDR3, FR4. The CDRs of a VH are termed HCDR1, HCDR2 and HCDR3, the CDRs of a VL are termed LCDR1, LCDR2 and LCDR3. The variable regions of the heavy and light chains contain a binding domain that interacts with an antigen. The constant regions of an antibody comprise the heavy chain constant region (CH) and the light chain constant region (CL), wherein CH can be further subdivided into constant domain CH1, a hinge region, and constant domains CH2 and CH3 (arranged from amino-terminus to carboxy-terminus in the following order: CH1, CH2, CH3).

The constant regions of the antibodies may mediate the binding of the immunoglobulin to host tissues or factors, including various cells of the immune system (e.g., effector cells) and the first component (Clq) of the classical complement system. Antibodies can be intact immunoglobulins derived from natural sources or from recombinant sources and can be immunoactive portions of intact immunoglobulins. Antibodies are typically tetramers of immunoglobulin molecules. Antibodies may exist in a variety of forms including, for example, polyclonal antibodies, monoclonal antibodies, Fv, Fab and F(ab)2, as well as single chain antibodies and humanized antibodies.

The term "immunoglobulin" relates to proteins of the immunoglobulin superfamily, preferably to antigen receptors such as antibodies or the B cell receptor (BCR). The immunoglobulins are characterized by a structural domain, i.e., the immunoglobulin domain, having a characteristic immunoglobulin (Ig) fold. The term encompasses membrane bound immunoglobulins as well as soluble immunoglobulins. Membrane bound immunoglobulins are also termed surface immunoglobulins or membrane immunoglobulins, which are generally part of the BCR. Soluble immunoglobulins are generally termed antibodies. Immunoglobulins generally comprise several chains, typically two identical heavy chains and two identical light chains which are linked via disulfide bonds. These chains are primarily composed of immunoglobulin domains, such as the V_{L} (variable light chain) domain, C_{L} (constant light chain) domain, V_{H} (variable heavy chain) domain, and the C_{H} (constant heavy chain) domains C_{H}1, C_{H}2, C_{H}3, and C_{H}4. There are five types of mammalian immunoglobulin heavy chains, i.e., α, δ, ε, γ, and µ which account for the different classes of antibodies, i.e., IgA, IgD, IgE, IgG, and IgM. As opposed to the heavy chains of soluble immunoglobulins, the heavy chains of membrane or surface immunoglobulins comprise a transmembrane domain and a short cytoplasmic domain at their carboxy-terminus. In mammals there are two types of light chains, i.e., lambda and kappa. The immunoglobulin chains comprise a variable region and a constant region. The constant region is essentially conserved within the different isotypes of the immunoglobulins, wherein the variable part is highly divers and accounts for antigen recognition.

An "antigen" according to the invention covers any substance that will elicit an immune response and/or any substance against which an immune response or an immune mechanism such as a cellular response is directed. This also includes situations wherein the antigen is processed into antigen peptides and an immune response or an immune mechanism is directed against one or more antigen peptides, in particular if presented in the context of MHC molecules. In particular, an "antigen" relates to any substance, preferably a peptide or protein, which reacts specifically with T lymphocytes (T cells). According to the present invention, the term "antigen" comprises any molecule which comprises at least one epitope such as a T cell epitope. Preferably, an antigen in the context of the present invention is a molecule which, optionally after processing, induces an immune reaction, which is preferably specific for the antigen (including cells expressing the antigen). In the context of the embodiments of the present invention, the antigen is preferably presented by a cell in the context of MHC molecules, which results in an immune response against the antigen.

As used herein, the term "self-antigen" refers to an antigen which is native to a mammal and which may be involved in the pathogenesis of an autoimmune disease.

The term "immunogenicity" relates to the relative effectivity of an antigen to induce an immune reaction.

The term "immunostimulatory" is used herein to refer to increasing overall immune response.

The term "immunosuppressive" is used herein to refer to reducing overall immune response. In some instances, it is desirable to induce an antigen specific immunosuppressive effect.

"Immune tolerance", "immunological tolerance", or simply "tolerance" describes a state of unresponsiveness of the immune system to substances or tissue that have the capacity to elicit an immune response. It contrasts with conventional immune-mediated elimination of foreign antigens. The mechanisms by which tolerance is established are distinct, but the resulting effect is similar.

An "antigen peptide" according to the invention preferably relates to a portion or fragment of an antigen which is capable of stimulating an immune response, preferably a cellular response against the antigen or cells characterized by expression of the antigen and preferably by presentation of the antigen. Preferably, an antigen peptide is capable of stimulating a cellular response against a cell characterized by presentation of an antigen with class I MHC and preferably is capable of stimulating an antigen-responsive cytotoxic T lymphocyte (CTL). Preferably, antigen peptides are MHC class I and/or class II presented peptides . Preferably, antigen peptides comprise an amino acid sequence substantially corresponding to the amino acid sequence of a fragment of an antigen. Preferably, said fragment of an antigen is an MHC class I and/or class II presented peptide.

A peptide which is suitable for binding to an MHC molecule, in particular a class I MHC molecule, preferably is 7-20 amino acids in length, more preferably 7-12 amino acids in length, more preferably 8-11 amino acids in length, in particular 9 or 10 amino acids in length.

In one embodiment, an antigen peptide when presented in the context of MHC such as MHC of antigen presenting cells is recognized by a T cell receptor. The antigen peptide if recognized by a T cell receptor may be able to induce in the presence of appropriate co-stimulatory signals, clonal expansion of the T cell carrying the T cell receptor specifically recognizing the antigen peptide. Preferably, antigen peptides, in particular if presented in the context of MHC molecules, are capable of stimulating an immune response, preferably a cellular response against the antigen from which they are derived or cells characterized by expression of the antigen and preferably characterized by presentation of the antigen.

The term "epitope" refers to an antigenic determinant in a molecule such as an antigen, i.e., to a part in or fragment of the molecule that is recognized by the immune system, for example, that is recognized by a T cell, in particular when presented in the context of MHC molecules. An epitope of a protein preferably comprises a continuous or discontinuous portion of said protein and is preferably between 5 and 100, preferably between 5 and 50, more preferably between 8 and 30, most preferably between 10 and 25 amino acids in length, for example, the epitope may be preferably 9, 10, 11, 12, 13, 14, 15, 16, 17, 18, 19, 20, 21, 22, 23, 24, or 25 amino acids in length. It is particularly preferred that the epitope in the context of the present invention is a T cell epitope.

Terms such as "epitope", "T cell epitope", "fragment of an antigen", "immunogenic peptide" and "antigen peptide" are used interchangeably herein and preferably relate to an incomplete representation of an antigen which is preferably capable of eliciting an immune response against the antigen or a cell expressing or comprising and preferably presenting the antigen. Preferably, the terms relate to an immunogenic portion of an antigen. Preferably, it is a portion of an antigen that is recognized (i.e., specifically bound) by a T cell receptor, in particular if presented in the context of MHC molecules. Certain preferred immunogenic portions bind to an MHC class I or class II molecule.

The term "target" shall mean an agent such as a cell or tissue which is a target for an immune response such as a cellular immune response. Targets include cells that present an antigen or an antigen epitope, i.e. a peptide fragment derived from an antigen. In one embodiment, the target cell is a cell expressing an antigen and preferably presenting said antigen with class I MHC.

The term "portion" refers to a fraction. With respect to a particular structure such as an amino acid sequence or protein the term "portion" thereof may designate a continuous or a discontinuous fraction of said structure.

The terms "part" and "fragment" are used interchangeably herein and refer to a continuous element. For example, a part of a structure such as an amino acid sequence or protein refers to a continuous element of said structure.

"Antigen processing" refers to the degradation of an antigen into procession products, which are fragments of said antigen (e.g., the degradation of a protein into peptides) and the association of one or more of these fragments (e.g., via binding) with MHC molecules for presentation by cells, preferably antigen presenting cells to specific T cells.

An antigen-presenting cell (APC) is a cell that displays antigen in the context of major histocompatibility complex (MHC) on its surface. T cells may recognize this complex using their T cell receptor (TCR). Antigen-presenting cells process antigens and present them to T cells. An antigen presenting cell includes, but is not limited to, monocytes/macrophages, B cells and dendritic cells (DCs).

Non-professional antigen-presenting cells do not constitutively express the MHC class II proteins required for interaction with naive T cells; these are expressed only upon stimulation of the non-professional antigen-presenting cells by certain cytokines such as IFNγ.

Professional antigen-presenting cells are very efficient at internalizing antigen, either by phagocytosis or by receptor-mediated endocytosis, and then displaying a fragment of the antigen, bound to a class II MHC molecule, on their membrane. The T cell recognizes and interacts with the antigen-class II MHC molecule complex on the membrane of the antigen-presenting cell. An additional co-stimulatory signal is then produced by the antigen-presenting cell, leading to activation of the T cell. The expression of co-stimulatory molecules is a defining feature of professional antigen-presenting cells.

The main types of professional antigen-presenting cells are dendritic cells, which have the broadest range of antigen presentation, and are probably the most important antigen-presenting cells, macrophages, B-cells, and certain activated epithelial cells.

Dendritic cells (DCs) are leukocyte populations that present antigens captured in peripheral tissues to T cells via both MHC class II and I antigen presentation pathways. It is well known that dendritic cells are potent inducers of immune responses and the activation of these cells is a critical step for the induction of immunity.

Dendritic cells are conveniently categorized as "immature" and "mature" cells, which can be used as a simple way to discriminate between two well characterized phenotypes. However, this nomenclature should not be construed to exclude all possible intermediate stages of differentiation.

Immature dendritic cells are characterized as antigen presenting cells with a high capacity for antigen uptake and processing, which correlates with the high expression of Fcy receptor and mannose receptor. The mature phenotype is typically characterized by a lower expression of these markers, but a high expression of cell surface molecules responsible for T cell activation such as class I and class II MHC, adhesion molecules (e. g. CD54 and CD11) and costimulatory molecules (e. g., CD40, CD80, CD86 and 4-1 BB).

Dendritic cell maturation is referred to as the status of dendritic cell activation at which such antigen-presenting dendritic cells lead to T cell priming, while presentation by immature dendritic cells results in tolerance. Dendritic cell maturation is chiefly caused by biomolecules with microbial features detected by innate receptors (bacterial DNA, viral RNA, endotoxin, etc.), pro-inflammatory cytokines (TNF, IL-1, IFNs), ligation of CD40 on the dendritic cell surface by CD40L, and substances released from cells undergoing stressful cell death. The dendritic cells can be derived by culturing bone marrow cells *in vitro* with cytokines, such as granulocyte-macrophage colony-stimulating factor (GM-CSF) and tumor necrosis factor alpha.

The term "immunoreactive cell" or "effector cell" in the context of the present invention relates to a cell which exerts effector functions during an immune reaction. An "immunoreactive cell" preferably is capable of binding an antigen or a cell characterized by expression and/or presentation of an antigen or an antigen peptide derived from an antigen and mediating an immune response. For example, such cells secrete cytokines and/or chemokines, kill microbes, secrete antibodies, recognize infected or cancerous cells, and optionally eliminate such cells. For example, immunoreactive cells comprise T cells (cytotoxic T cells, helper T cells, tumor infiltrating T cells), B cells, natural killer cells, neutrophils, macrophages, and dendritic cells. Preferably, in the context of the present invention, "immunoreactive cells" are T cells, preferably CD4⁺ and/or CD8⁺ T cells.

Preferably, an "immunoreactive cell" recognizes an antigen or an antigen peptide derived from an antigen with some degree of specificity, in particular if presented in the context of MHC molecules such as on the surface of antigen presenting cells or diseased cells such as tumor cells. Preferably, said recognition enables the cell that recognizes an antigen or an antigen peptide derived from said antigen to be responsive or reactive. If the cell is a helper T cell (CD4⁺ T cell) bearing receptors that recognize an antigen or an antigen peptide derived from an antigen in the context of MHC class II molecules such responsiveness or reactivity may involve the release of cytokines and/or the activation of CD8⁺ lymphocytes (CTLs) and/or B-cells. If the cell is a CTL such responsiveness or reactivity may involve the elimination of cells presented in the context of MHC class I molecules, i.e., cells characterized by presentation of an antigen with class I MHC, for example, via apoptosis or perforin-mediated cell lysis. According to the invention, CTL responsiveness may include sustained calcium flux, cell division, production of cytokines such as IFN-γ and TNF-α, up-regulation of activation markers such as CD44 and CD69, and specific cytolytic killing of antigen expressing target cells. CTL responsiveness may also be determined using an artificial reporter that accurately indicates CTL responsiveness. Such CTL that recognizes an antigen or an antigen peptide derived from an antigen and are responsive or reactive are also termed "antigen-responsive CTL" herein. If the cell is a B cell such responsiveness may involve the release of immunoglobulins.

The term "T cell" or "T lymphocyte" relates to thymus-derived cells that participate in a variety of cell-mediated immune reactions and includes T helper cells (CD4+ T cells) and cytotoxic T cells (CTLs, CD8+ T cells) which comprise cytolytic T cells.

T cells belong to a group of white blood cells known as lymphocytes, and play a central role in cell-mediated immunity. They can be distinguished from other lymphocyte types, such as B cells and natural killer cells by the presence of a special receptor on their cell surface called T cell receptor (TCR). The thymus is the principal organ responsible for the maturation of T cells. Several different subsets of T cells have been discovered, each with a distinct function.

T helper cells assist other white blood cells in immunologic processes, including maturation of B cells into plasma cells and activation of cytotoxic T cells and macrophages, among other functions. These cells are also known as CD4+ T cells because they express the CD4 protein on their surface. Helper T cells become activated when they are presented with peptide antigens by MHC class II molecules that are expressed on the surface of antigen presenting cells (APCs). Once activated, they divide rapidly and secrete small proteins called cytokines that regulate or assist in the active immune response.

Cytotoxic T cells destroy virally infected cells and tumor cells, and are also implicated in transplant rejection. These cells are also known as CD8+ T cells since they express the CD8 glycoprotein at their surface. These cells recognize their targets by binding to antigen associated with MHC class I, which is present on the surface of nearly every cell of the body.

A majority of T cells have a T cell receptor (TCR) existing as a complex of several proteins. The actual T cell receptor is composed of two separate peptide chains, which are produced from the independent T cell receptor alpha and beta (TCRα and TCRβ) genes and are called α- and β-TCR chains. γδ T cells (gamma delta T cells) represent a small subset of T cells that possess a distinct T cell receptor (TCR) on their surface. However, in γδ T cells, the TCR is made up of one γ-chain and one δ-chain. This group of T cells is much less common (2% of total T cells) than the αβ T cells.

The structure of the T cell receptor is very similar to immunoglobulin Fab fragments, which are regions defined as the combined light and heavy chain of an antibody arm. Each chain of the TCR is a member of the immunoglobulin superfamily and possesses one N-terminal immunoglobulin (Ig)-variable (V) domain, one Ig-constant (C) domain, a transmembrane/cell membrane-spanning region, and a short cytoplasmic tail at the C-terminal end. The variable domain of both the TCR α-chain and β-chain have three hypervariable or complementarity determining regions (CDRs), whereas the variable region of the β-chain has an additional area of hypervariability (HV4) that does not normally contact antigen and therefore is not considered a CDR. CDR3 is the main CDR responsible for recognizing processed antigen, although CDR1 of the α-chain has also been shown to interact with the N-terminal part of the antigenic peptide, whereas CDR1 of the β-chain interacts with the C-terminal part of the peptide. CDR2 is thought to recognize the MHC. CDR4 of the β-chain is not thought to participate in antigen recognition, but has been shown to interact with superantigens. The constant domain of the TCR domain consists of short connecting sequences in which a cysteine residue forms disulfide bonds, which forms a link between the two chains.

The term "peripheral blood mononuclear cell" or "PBMC" relates to a peripheral blood cell having a round nucleus. These cells consist of lymphocytes (T cells, B cells, NK cells) and monocytes, whereas erythrocytes and platelets have no nuclei, and granulocytes (neutrophils, basophils, and eosinophils) have multi-lobed nuclei. These cells can be extracted from whole blood using ficoll and gradient centrifugation, which will separate the blood into a top layer of plasma, followed by a layer of PBMCs and a bottom fraction of polymorphonuclear cells (such as neutrophils and eosinophils) and erythrocytes.

The term "major histocompatibility complex" and the abbreviation "MHC" include MHC class I and MHC class II molecules and relate to a complex of genes which occurs in all vertebrates. MHC proteins or molecules are important for signaling between lymphocytes and antigen presenting cells or diseased cells in immune reactions, wherein the MHC proteins or molecules bind peptides and present them for recognition by T cell receptors. The proteins encoded by the MHC are expressed on the surface of cells, and display both self antigens (peptide fragments from the cell itself) and nonself antigens (e.g., fragments of invading microorganisms) to a T cell.

The MHC region is divided into three subgroups, class I, class II, and class III. MHC class I proteins contain an α-chain and β2-microglobulin (not part of the MHC encoded by chromosome 15). They present antigen fragments to cytotoxic T cells. On most immune system cells, specifically on antigen-presenting cells, MHC class II proteins contain α- and β-chains and they present antigen fragments to T-helper cells. MHC class III region encodes for other immune components, such as complement components and some that encode cytokines.

In humans, genes in the MHC region that encode antigen-presenting proteins on the cell surface are referred to as human leukocyte antigen (HLA) genes. However the abbreviation MHC is often used to refer to HLA gene products. HLA genes include the nine so-called classical MHC genes: HLA-A, HLA-B, HLA-C, HLA-DPA1, HLA-DPB1, HLA-DQA1, HLA-DQB1, HLA-DRA, and HLA-DRB1.

In one preferred embodiment of all aspects of the invention an MHC molecule is an HLA molecule.

The term "immune effector functions" or "effector functions" in the context of the present invention includes any functions mediated by components of the immune system that result, for example, in the killing of cells. Preferably, the immune effector functions in the context of the present invention are T cell mediated effector functions. Such functions comprise in the case of a helper T cell (CD4⁺ T cell) the recognition of an antigen or an antigen peptide derived from an antigen in the context of MHC class II molecules by T cell receptors, the release of cytokines and/or the activation of CD8⁺ lymphocytes (CTLs) and/or B-cells, and in the case of CTL the recognition of an antigen or an antigen peptide derived from an antigen in the context of MHC class I molecules by T cell receptors, the elimination of cells presented in the context of MHC class I molecules, i.e., cells characterized by presentation of an antigen with class I MHC, for example, via apoptosis or perforin-mediated cell lysis, production of cytokines such as IFN-γ and TNF-α, and specific cytolytic killing of antigen expressing target cells.

A "nucleic acid" is according to the invention preferably deoxyribonucleic acid (DNA) or ribonucleic acid (RNA), more preferably RNA, most preferably *in vitro* transcribed RNA (IVT RNA) or synthetic RNA. Nucleic acids include according to the invention genomic DNA, cDNA, mRNA, recombinantly produced and chemically synthesized molecules. According to the invention, a nucleic acid may be present as a single-stranded or doublestranded and linear or covalently circularly closed molecule. A nucleic acid can, according to the invention, be isolated. The term "isolated nucleic acid" means, according to the invention, that the nucleic acid (i) was amplified *in vitro,* for example via polymerase chain reaction (PCR), (ii) was produced recombinantly by cloning, (iii) was purified, for example, by cleavage and separation by gel electrophoresis, or (iv) was synthesized, for example, by chemical synthesis. A nucleic can be employed for introduction into, i.e. transfection of, cells, for example, in the form of RNA which can be prepared by *in vitro* transcription from a DNA template. The RNA can moreover be modified before application by stabilizing sequences, capping, and polyadenylation.

The nucleic acids described herein may be comprised in a vector which can be used to deliver a nucleic acid to the interior of a cell. The term "vector" as used herein includes any vectors known to the skilled person including plasmid vectors, cosmid vectors, phage vectors such as lambda phage, viral vectors such as adenoviral or baculoviral vectors, retro- or lentiviral vectors, transposons or artificial chromosome vectors such as bacterial artificial chromosomes (BAC), yeast artificial chromosomes (YAC), or P1 artificial chromosomes (PAC). Said vectors include expression as well as cloning vectors. Expression vectors comprise plasmids as well as viral vectors and generally contain a desired coding sequence and appropriate DNA sequences necessary for the expression of the operably linked coding sequence in a particular host organism (e.g., bacteria, yeast, plant, insect, or mammal) or in *in vitro* expression systems. Cloning vectors are generally used to engineer and amplify a certain desired DNA fragment and may lack functional sequences needed for expression of the desired DNA fragments.

The tolerogenic or immunosuppressive cells described herein can be generated either *in vitro* or *in vivo* by transfecting or transducing the cells with a vector that results in increased expression of CFLAR. Any of a variety of methods well known to one of skill in the art can be used to transfect or transduce the cells.

The nucleic acid encoding CFLAR can be cloned into a number of types of vectors. However, the present invention should not be construed to be limited to any particular vector. Instead, the present invention should be construed to encompass a wide plethora of vectors which are readily available and well-known in the art. In specific embodiments, the vector is selected from the group consisting of a viral vector, a bacterial vector, and a mammalian cell vector. Many such systems are commercially and widely available.

The vector may be provided to a cell in the form of a viral vector. Viral vector technology is well known in the art. Viruses, which are useful as vectors include, but are not limited to, retroviruses, adenoviruses, adeno-associated viruses, herpes viruses, and lentiviruses. Preferably, the virus is helper-dependent adenovirus (HD-Ad). In general, a suitable vector contains an origin of replication functional in at least one organism, a promoter sequence, convenient restriction endonuclease sites, and one or more selectable markers.

Those of skill in the art of molecular biology generally know how to use promoters, enhancers, and cell type combinations for protein expression. The promoters employed may be constitutive, tissue-specific, inducible, and/or useful under the appropriate conditions to direct high level expression of the introduced nucleic acid segment encoding CFLAR. The promoter may be heterologous or endogenous. Constitutive promoter sequences which may be used according to the invention, include, but are not limited to the immediate early cytomegalovirus (CMV) promoter sequence, the simian virus 40 (SV40) early promoter, mouse mammary tumor virus (MMTV), human immunodeficiency virus (HIV) long terminal repeat (LTR) promoter, Moloney virus promoter, the avian leukemia virus promoter, Epstein-Barr virus immediate early promoter, Rous sarcoma virus promoter, as well as human gene promoters such as, but not limited to, the actin promoter, the myosin promoter, the hemoglobin promoter, and the muscle creatine promoter. Further, the invention should not be limited to the use of constitutive promoters. Inducible promoters are also contemplated as part of the invention. The use of an inducible promoter in the invention provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter. Further, the invention includes the use of a tissue specific promoter, which promoter is active only in a desired tissue. Tissue specific promoters are well known in the art and include, but are not limited to, the HER-2 promoter and the PSA associated promoter sequences.

In order to assess the expression of CFLAR, the expression vector to be introduced into a cell can also contain either a selectable marker gene or a reporter gene or both to facilitate identification and selection of expressing cells from the population of cells sought to be transfected or infected through viral vectors. In other embodiments, the selectable marker may be carried on a separate piece of DNA and used in a co-transfection procedure. Both selectable markers and reporter genes may be flanked with appropriate regulatory sequences to enable expression in the host cells. Useful selectable markers are known in the art and include, for example, antibiotic-resistance genes, such as neo and the like. Reporter genes are used for identifying potentially transfected cells and for evaluating the functionality of regulatory sequences. Reporter genes that encode for easily assayable proteins are well known in the art. In general, a reporter gene is a gene that is not present in or expressed by the recipient organism or tissue and that encodes a protein whose expression is manifested by some easily detectable property, e.g., enzymatic activity. Expression of the reporter gene is assayed at a suitable time after the nucleic acid has been introduced into the recipient cells. Suitable reporter genes may include genes encoding luciferase, beta-galactosidase, chloramphenicol acetyl transferase, secreted alkaline phosphatase, or the green fluorescent protein gene.

The vector can be readily introduced into a host cell by any method in the art. For example, the expression vector can be transferred into a host cell by physical, chemical or biological means. Physical methods for introducing a nucleic acid into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York), and in Ausubel et al. (1997, Current Protocols in Molecular Biology, John Wiley & Sons, New York).

Biological methods for introducing a nucleic acid of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like.

Chemical means for introducing a nucleic acid into a host cell include colloidal dispersion systems, such as macromolecule complexes, nanocapsules, microspheres, beads, and lipid-based systems including oil-in-water emulsions, micelles, mixed micelles, and liposomes. A preferred colloidal system for use as a delivery vehicle *in vitro* and *in vivo* is a liposome (i.e., an artificial membrane vesicle). The preparation and use of such systems is well known in the art.

Regardless of the method used to introduce exogenous nucleic acids into a host cell or otherwise increase the cellular level of CFLAR in a cell, in order to confirm the presence and/or amount of CFLAR or its encoding nucleic acid in the host cell, a variety of assays may be performed. Such assays include, for example, Southern and Northern blotting, RT-PCR and PCR and assays for detecting the presence or absence of a particular peptide, e.g., by immunological means (ELISAs and Western blots).

In the context of the present invention, the term "DNA" relates to a molecule which comprises deoxyribonucleotide residues and preferably is entirely or substantially composed of deoxyribonucleotide residues. "Deoxyribonucleotide" relates to a nucleotide which lacks a hydroxyl group at the 2'-position of a β-D-ribofuranosyl group. The term "DNA" comprises isolated DNA such as partially or completely purified DNA, essentially pure DNA, synthetic DNA, and recombinantly generated DNA and includes modified DNA which differs from naturally occurring DNA by addition, deletion, substitution and/or alteration of one or more nucleotides. Such alterations can include addition of non-nucleotide material, such as to the end(s) of a DNA or internally, for example at one or more nucleotides of the DNA. Nucleotides in DNA molecules can also comprise non-standard nucleotides, such as non-naturally occurring nucleotides or chemically synthesized nucleotides. These altered DNAs can be referred to as analogs or analogs of naturally-occurring DNA.

In the context of the present invention, the term "RNA" relates to a molecule which comprises ribonucleotide residues and preferably being entirely or substantially composed of ribonucleotide residues. "Ribonucleotide" relates to a nucleotide with a hydroxyl group at the 2'-position of a β-D-ribofuranosyl group. The term includes double stranded RNA, single stranded RNA, isolated RNA such as partially or completely purified RNA, essentially pure RNA, synthetic RNA, recombinantly produced RNA, as well as modified RNA that differs from naturally occurring RNA by the addition, deletion, substitution and/or alteration of one or more nucleotides. Such alterations can include addition of non-nucleotide material, such as to the end(s) of a RNA or internally, for example at one or more nucleotides of the RNA. Nucleotides in RNA molecules can also comprise non-standard nucleotides, such as non-naturally occurring nucleotides or chemically synthesized nucleotides or deoxynucleotides. These altered RNAs can be referred to as analogs or analogs of naturally-occurring RNA. According to the present invention, the term "RNA" includes and preferably relates to "mRNA" which means "messenger RNA" and relates to a transcript which may be produced using DNA as template and encodes a peptide or protein. mRNA typically comprises a 5' non translated region (5'-UTR), a protein or peptide coding region and a 3' non translated region (3'-UTR). mRNA has a limited halftime in cells and *in vitro.* Preferably, mRNA is produced by *in vitro* transcription using a DNA template. In one embodiment of the invention, the RNA is obtained by *in vitro* transcription or chemical synthesis. The *in vitro* transcription methodology is known to the skilled person. For example, there is a variety of *in vitro* transcription kits commercially available.

According to the invention, the stability and translation efficiency of RNA may be modified as required. For example, RNA may be stabilized and its translation increased by one or more modifications having a stabilizing effects and/or increasing translation efficiency of RNA. In order to increase expression of the RNA used according to the present invention, it may be modified within the coding region, i.e. the sequence encoding the expressed peptide or protein, preferably without altering the sequence of the expressed peptide or protein, so as to increase the GC-content to increase mRNA stability and to perform a codon optimization and, thus, enhance translation in cells.

The term "modification" in the context of the RNA used in the present invention includes any modification of an RNA which is not naturally present in said RNA.

In one embodiment of the invention, the RNA used according to the invention does not have uncapped 5'-triphosphates. Removal of such uncapped 5'-triphosphates can be achieved by treating RNA with a phosphatase.

The RNA according to the invention may have modified ribonucleotides in order to increase its stability and/or decrease cytotoxicity. For example, in one embodiment, in the RNA used according to the invention 5-methylcytidine is substituted partially or completely, preferably completely, for cytidine. Alternatively or additionally, in one embodiment, in the RNA used according to the invention pseudouridine is substituted partially or completely, preferably completely, for uridine.

In one embodiment, the term "modification" relates to providing an RNA with a 5'-cap or 5'-cap analog. The term "5'-cap" refers to a cap structure found on the 5'-end of an mRNA molecule and generally consists of a guanosine nucleotide connected to the mRNA via an unusual 5' to 5' triphosphate linkage. In one embodiment, this guanosine is methylated at the 7-position. The term "conventional 5'-cap" refers to a naturally occurring RNA 5'-cap, preferably to the 7-methylguanosine cap (m⁷G). In the context of the present invention, the term "5'-cap" includes a 5'-cap analog that resembles the RNA cap structure and is modified to possess the ability to stabilize RNA and/or enhance translation of RNA if attached thereto, preferably *in vivo* and/or in a cell.

The RNA may comprise further modifications. For example, a further modification of the RNA used in the present invention may be an extension or truncation of the naturally occurring poly(A) tail or an alteration of the 5'- or 3'-untranslated regions (UTR) such as introduction of a UTR which is not related to the coding region of said RNA, for example, the exchange of the existing 3'-UTR with or the insertion of one or more, preferably two copies of a 3'-UTR derived from a globin gene, such as alpha2-globin, alphal-globin, beta-globin, preferably beta-globin, more preferably human beta-globin.

RNA having an unmasked poly-A sequence is translated more efficiently than RNA having a masked poly-A sequence. The term "poly(A) tail" or "poly-A sequence" relates to a sequence of adenyl (A) residues which typically is located on the 3'-end of a RNA molecule and "unmasked poly-A sequence" means that the poly-A sequence at the 3' end of an RNA molecule ends with an A of the poly-A sequence and is not followed by nucleotides other than A located at the 3' end, i.e. downstream, of the poly-A sequence. Furthermore, a long poly-A sequence of about 120 base pairs results in an optimal transcript stability and translation efficiency of RNA.

Therefore, in order to increase stability and/or expression of the RNA used according to the present invention, it may be modified so as to be present in conjunction with a poly-A sequence, preferably having a length of 10 to 500, more preferably 30 to 300, even more preferably 65 to 200 and especially 100 to 150 adenosine residues. In an especially preferred embodiment the poly-A sequence has a length of approximately 120 adenosine residues. To further increase stability and/or expression of the RNA used according to the invention, the poly-A sequence can be unmasked.

The term "stability" of RNA relates to the "half-life" of RNA. "Half-life" relates to the period of time which is needed to eliminate half of the activity, amount, or number of molecules. In the context of the present invention, the half-life of an RNA is indicative for the stability of said RNA. The half-life of RNA may influence the "duration of expression" of the RNA. It can be expected that RNA having a long half-life will be expressed for an extended time period.

Of course, if according to the present invention it is desired to decrease stability and/or translation efficiency of RNA, it is possible to modify RNA so as to interfere with the function of elements as described above increasing the stability and/or translation efficiency of RNA.

"Encoding" refers to the inherent property of specific sequences of nucleotides in a nucleic acid to serve as templates for synthesis of other polymers and macromolecules in biological processes having either a defined sequence of nucleotides or a defined sequence of amino acids. Thus, a nucleic acid encodes a protein if expression (translation and optionally transcription) of the nucleic acid produces the protein in a cell or other biological system.

The term "expression" is used according to the invention in its most general meaning and comprises the production of RNA and/or peptides or polypeptides, e.g. by transcription and/or translation. With respect to RNA, the term "expression" or "translation" relates in particular to the production of peptides or polypeptides. It also comprises partial expression of nucleic acids. Moreover, expression can be transient or stable.

In the context of the present invention, the term "transcription" relates to a process, wherein the genetic code in a DNA sequence is transcribed into RNA. Subsequently, the RNA may be translated into protein. According to the present invention, the term "transcription" comprises *"in vitro* transcription", wherein the term *"in vitro* transcription" relates to a process wherein RNA, in particular mRNA, is *in vitro* synthesized in a cell-free system, preferably using appropriate cell extracts. Preferably, cloning vectors are applied for the generation of transcripts. These cloning vectors are generally designated as transcription vectors and are according to the present invention encompassed by the term "vector". According to the present invention, the RNA used in the present invention preferably is *in vitro* transcribed RNA (IVT-RNA) and may be obtained by *in vitro* transcription of an appropriate DNA template. The promoter for controlling transcription can be any promoter for any RNA polymerase. Particular examples of RNA polymerases are the T7, T3, and SP6 RNA polymerases. Preferably, the *in vitro* transcription according to the invention is controlled by a T7 or SP6 promoter. A DNA template for *in vitro* transcription may be obtained by cloning of a nucleic acid, in particular cDNA, and introducing it into an appropriate vector for *in vitro* transcription. The cDNA may be obtained by reverse transcription of RNA.

The term "translation" according to the invention relates to the process in the ribosomes of a cell by which a strand of messenger RNA directs the assembly of a sequence of amino acids to make a peptide or polypeptide.

Expression control sequences or regulatory sequences, which according to the invention may be linked functionally with a nucleic acid, can be homologous or heterologous with respect to the nucleic acid. A coding sequence and a regulatory sequence are linked together "functionally" if they are bound together covalently, so that the transcription or translation of the coding sequence is under the control or under the influence of the regulatory sequence. If the coding sequence is to be translated into a functional protein, with functional linkage of a regulatory sequence with the coding sequence, induction of the regulatory sequence leads to a transcription of the coding sequence, without causing a reading frame shift in the coding sequence or inability of the coding sequence to be translated into the desired protein or peptide.

The term "expression control sequence" or "regulatory sequence" comprises, according to the invention, promoters, ribosome-binding sequences and other control elements, which control the transcription of a nucleic acid or the translation of the derived RNA. In certain embodiments of the invention, the regulatory sequences can be controlled. The precise structure of regulatory sequences can vary depending on the species or depending on the cell type, but generally comprises 5'-untranscribed and 5'- and 3'-untranslated sequences, which are involved in the initiation of transcription or translation, such as TATA-box, capping-sequence, CAAT-sequence and the like. In particular, 5'-untranscribed regulatory sequences comprise a promoter region that includes a promoter sequence for transcriptional control of the functionally bound gene. Regulatory sequences can also comprise enhancer sequences or upstream activator sequences.

According to the invention it is preferred that a nucleic acid such as RNA encoding a peptide or protein once taken up by or introduced, i.e. transfected or transduced, into a cell which cell may be present *in vitro* or in a subject results in expression of said peptide or protein. The cell may express the encoded peptide or protein intracellularly (e.g. in the cytoplasm and/or in the nucleus), may secrete the encoded peptide or protein, or may express it on the surface.

According to the invention, terms such as "nucleic acid expressing" and "nucleic acid encoding" or similar terms are used interchangeably herein and with respect to a particular peptide or polypeptide mean that the nucleic acid, if present in the appropriate environment, preferably within a cell, can be expressed to produce said peptide or polypeptide.

According to the invention, any means can be used to engineer cells so as to increase the cellular level of CFLAR. In one embodiment, cells are engineered so as to increase the cellular level of CFLAR by transfecting the cells with nucleic acid encoding a CFLAR polypeptide.

Terms such as "transferring", "introducing", "transfecting" or "transducing" are used interchangeably herein and relate to the introduction of nucleic acids, in particular exogenous or heterologous nucleic acids, such as RNA into a cell. According to the present invention, the cell can be present *in vitro* or *in vivo,* e.g. the cell can form part of an organ, a tissue and/or an organism. According to the invention, transfection can be transient or stable. For some applications of transfection, it is sufficient if the transfected genetic material is only transiently expressed. Since the nucleic acid introduced in the transfection process is usually not integrated into the nuclear genome, the foreign nucleic acid will be diluted through mitosis or degraded. Cell lines allowing episomal amplification of nucleic acids greatly reduce the rate of dilution. If it is desired that the transfected nucleic acid actually remains in the genome of the cell and its daughter cells, a stable transfection must occur. RNA can be transfected into cells to transiently express its coded protein.

The term "cellular FLICE [Fas-associated death domain (FADD)-like IL-1β-converting enzyme]-inhibitory protein", "cellular FLICE-inhibitory protein", "CFLAR" or "c-FLIP" is a master anti-apoptotic regulator and resistance factor that suppresses tumor necrosis factor-α (TNF-α), Fas-L, and TNF-related apoptosis-inducing ligand (TRAIL)-induced apoptosis, as well as apoptosis triggered by chemotherapy agents in malignant cells. CFLAR is expressed as long (CFLAR_{L}), short (CFLARs), and CFLAR_{R} splice variants in human cells. CFLAR binds to FADD and/or caspase-8 or -10 and TRAIL receptor 5 (DR5) in a ligand-dependent and -independent fashion and forms an apoptosis inhibitory complex (AIC). This interaction in turn prevents death-inducing signaling complex (DISC) formation and subsequent activation of the caspase cascade. CFLAR_{L} and CFLARs are also known to have multifunctional roles in various signaling pathways, as well as activating and/or upregulating several cytoprotective and pro-survival signaling proteins including Akt, ERK, and NF-kB. According to the invention, the term "CFLAR" includes any variants, in particular mutants, splice variants, conformations, isoforms, allelic variants, species variants and species homologs, in particular those which are naturally present, and comprises CFLAR_{L}, CFLARs, CFLAR_{R} and v-CFLAR.

The term "CFLAR_{L}" preferably relates to a protein comprising the amino acid sequence according to SEQ ID NO: 1 of the sequence listing or a variant of said amino acid sequence. In one embodiment, the amino acid sequence according to SEQ ID NO: 1 of the sequence listing is encoded by the nucleic acid sequence according to SEQ ID NO: 2 of the sequence listing.

The term "CFLAR_{S}" preferably relates to a protein comprising the amino acid sequence according to SEQ ID NO: 3 of the sequence listing or a variant of said amino acid sequence. In one embodiment, the amino acid sequence according to SEQ ID NO: 3 of the sequence listing is encoded by the nucleic acid sequence according to SEQ ID NO: 4 of the sequence listing.

The term "CFLAR_{R}" preferably relates to a protein comprising the amino acid sequence according to SEQ ID NO: 5 of the sequence listing or a variant of said amino acid sequence. In one embodiment, the amino acid sequence according to SEQ ID NO: 5 of the sequence listing is encoded by the nucleic acid sequence according to SEQ ID NO: 6 of the sequence listing.

The term "v-CFLAR" preferably relates to a protein comprising the amino acid sequence according to SEQ ID NO: 7 of the sequence listing or a variant of said amino acid sequence. In one embodiment, the amino acid sequence according to SEQ ID NO: 7 of the sequence listing is encoded by the nucleic acid sequence according to SEQ ID NO: 8 of the sequence listing.

According to the present invention, the term "peptide" refers to substances comprising two or more, preferably 3 or more, preferably 4 or more, preferably 6 or more, preferably 8 or more, preferably 10 or more, preferably 13 or more, preferably 16 more, preferably 21 or more and up to preferably 8, 10, 20, 30, 40 or 50, in particular 100 amino acids joined covalently by peptide bonds.

The term "protein" refers to large peptides, i.e. polypeptides, preferably to peptides with more than 100 amino acid residues, but in general the terms "peptide", "polypeptide" and "protein" are synonyms and are used interchangeably herein.

The present invention also includes "variants" of the peptides, proteins, or amino acid sequences described herein.

For the purposes of the present invention, "variants" of an amino acid sequence comprise amino acid insertion variants, amino acid addition variants, amino acid deletion variants and/or amino acid substitution variants.

Amino acid insertion variants comprise insertions of single or two or more amino acids in a particular amino acid sequence. In the case of amino acid sequence variants having an insertion, one or more amino acid residues are inserted into a particular site in an amino acid sequence, although random insertion with appropriate screening of the resulting product is also possible.

Amino acid addition variants comprise amino- and/or carboxy-terminal fusions of one or more amino acids, such as 1, 2, 3, 5, 10, 20, 30, 50, or more amino acids.

Amino acid deletion variants are characterized by the removal of one or more amino acids from the sequence, such as by removal of 1, 2, 3, 5, 10, 20, 30, 50, or more amino acids. The deletions may be in any position of the protein. Amino acid deletion variants that comprise the deletion at the N-terminal and/or C-terminal end of the protein are also called N-terminal and/or C-terminal truncation variants.

Amino acid substitution variants are characterized by at least one residue in the sequence being removed and another residue being inserted in its place. Preference is given to the modifications being in positions in the amino acid sequence which are not conserved between homologous proteins or peptides and/or to replacing amino acids with other ones having similar properties. Preferably, amino acid changes in protein variants are conservative amino acid changes, i.e., substitutions of similarly charged or uncharged amino acids. A conservative amino acid change involves substitution of one of a family of amino acids which are related in their side chains. Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids.

Preferably the degree of similarity, preferably identity between a given amino acid sequence and an amino acid sequence which is a variant of said given amino acid sequence will be at least about 60%, 65%, 70%, 80%, 81%, 82%, 83%, 84%, 84%, 85%, 86%, 87%, 88%, 89%, 90%, 91%, 92%, 93%, 94%, 95%, 96%, 97%, 98%, or 99%. The degree of similarity or identity is given preferably for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given preferably for at least about 20, at least about 40, at least about 60, at least about 80, at least about 100, at least about 120, at least about 140, at least about 160, at least about 180, or about 200 amino acids, preferably continuous amino acids. The degree of similarity or identity is given preferably for a segment of at least 80, at least 100, at least 120, at least 150, at least 180, at least 200 or at least 250 amino acids. In preferred embodiments, the degree of similarity or identity is given for the entire length of the reference amino acid sequence. The alignment for determining sequence similarity, preferably sequence identity can be done with art known tools, preferably using the best sequence alignment, for example, using Align, using standard settings, preferably EMBOSS::needle, Matrix: Blosum62, Gap Open 10.0, Gap Extend 0.5.

"Sequence similarity" indicates the percentage of amino acids that either are identical or that represent conservative amino acid substitutions. "Sequence identity" between two amino acid sequences indicates the percentage of amino acids that are identical between the sequences.

The term "percentage identity" is intended to denote a percentage of amino acid residues which are identical between the two sequences to be compared, obtained after the best alignment, this percentage being purely statistical and the differences between the two sequences being distributed randomly and over their entire length. Sequence comparisons between two amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Neddleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

The percentage identity is calculated by determining the number of identical positions between the two sequences being compared, dividing this number by the number of positions compared and multiplying the result obtained by 100 so as to obtain the percentage identity between these two sequences.

Homologous amino acid sequences exhibit according to the invention at least 40%, in particular at least 50%, at least 60%, at least 70%, at least 80%, at least 90% and preferably at least 95%, at least 98 or at least 99% identity of the amino acid residues.

The amino acid sequence variants described herein may readily be prepared by the skilled person, for example, by recombinant DNA manipulation. The manipulation of DNA sequences for preparing peptides or proteins having substitutions, additions, insertions or deletions, is described in detail in Sambrook et al. (1989), for example. Furthermore, the peptides and amino acid variants described herein may be readily prepared with the aid of known peptide synthesis techniques such as, for example, by solid phase synthesis and similar methods.

The invention includes derivatives of the peptides or proteins described herein which are comprised by the terms "peptide" and "protein". According to the invention, "derivatives" of proteins and peptides are modified forms of proteins and peptides. Such modifications include any chemical modification and comprise single or multiple substitutions, deletions and/or additions of any molecules associated with the protein or peptide, such as carbohydrates, lipids and/or proteins or peptides. In one embodiment, "derivatives" of proteins or peptides include those modified analogs resulting from glycosylation, acetylation, phosphorylation, amidation, palmitoylation, myristoylation, isoprenylation, lipidation, alkylation, derivatization, introduction of protective/blocking groups, proteolytic cleavage or binding to an antibody or to another cellular ligand. The term "derivative" also extends to all functional chemical equivalents of said proteins and peptides. Preferably, a modified peptide has increased stability and/or increased immunogenicity.

According to the invention, a variant or derivative of a peptide or protein preferably has a functional property of the peptide or protein from which it has been derived. Preferably, a variant or derivative of a CFLAR protein has the same or similar property as the CFLAR protein from which it has been derived.

The term "derived" means according to the invention that a particular entity, in particular a particular sequence, is present in the object from which it is derived, in particular an organism or molecule. In the case of amino acid sequences, especially particular sequence regions, "derived" in particular means that the relevant amino acid sequence is derived from an amino acid sequence in which it is present.

The term "cell" or "host cell" preferably is an intact cell, i.e. a cell with an intact membrane that has not released its normal intracellular components such as enzymes, organelles, or genetic material. An intact cell preferably is a viable cell, i.e. a living cell capable of carrying out its normal metabolic functions. Preferably said term relates according to the invention to any cell which can be transformed or transfected with an exogenous nucleic acid. The term "cell" includes according to the invention prokaryotic cells (e.g., E. coli) or eukaryotic cells (e.g., dendritic cells, B cells, CHO cells, COS cells, K562 cells, HEK293 cells, HELA cells, yeast cells, and insect cells). The exogenous nucleic acid may be found inside the cell (i) freely dispersed as such, (ii) incorporated in a recombinant vector, or (iii) integrated into the host cell genome or mitochondrial DNA. Mammalian cells are particularly preferred, such as cells from humans, mice, hamsters, pigs, goats, and primates. The cells may be derived from a large number of tissue types and include primary cells and cell lines.

A cell which comprises a nucleic acid, e.g. which has been transfected with a nucleic acid, preferably expresses the peptide or protein encoded by the nucleic acid.

The term "expansion" refers to a process wherein a specific entity is multiplied. In one embodiment of the present invention, the term is used in the context of an immunological response in which lymphocytes are stimulated by an antigen, proliferate, and the specific lymphocyte recognizing said antigen is amplified. Preferably, clonal expansion leads to differentiation of the lymphocytes.

The tolerogenic or immunosuppressive cells described herein (either generated *in vitro* for administration to a subject or generated *in situ* in a subject, for example, by administration of a nucleic acid encoding CFLAR) are useful in methods of suppressing an immune response in a mammal for the treatment or prevention of an autoimmune disease or an inflammatory disease, or transplantation rejection including organ, tissue, cell, and/or protein transplantation. Without wishing to be bound to any particular theory, the tolerogenic or immunosuppressive cells that are administered to the patient or are formed *in situ* in the patient inhibit the activation and proliferation of the patient's T cells.

The tolerogenic or immunosuppressive cells are useful in avoiding or suppressing side effects resulting from the patient's immune response mounted against protein administered to the patient (optionally through expression of a nucleic acid administered to a patient), which therefore decreases the efficacy and safety of the protein. Protein therapeutics include, but is not limited to monoclonal antibodies, enzymes, cytokines, and toxins.

Furthermore, the tolerogenic or immunosuppressive cells are useful for reducing and/or eliminating an immune response to a transplant in a recipient by administering to or generating in the recipient of the transplant an amount of tolerogenic or immunosuppressive cells effective to reduce or inhibit host rejection of the transplant. The transplant can include a biocompatible lattice or a donor tissue, organ, cell or molecule, to be transplanted. An example of a transplant may include but is not limited to skin cells or tissue, bone marrow, and solid organs such as heart, pancreas, kidney, lung and liver. In some instances, the transplant is a nucleic acid or a protein. Based upon the disclosure provided herein, myeloid cells or progenitor cells thereof can be obtained from any source, for example, from the transplantat donor, the transplant recipient or an otherwise unrelated source (a different individual or species altogether). The tolerogenic or immunosuppressive cells are preferably administered to or generated in the recipient prior to, or contemporaneously with a transplant to reduce and/or eliminate host rejection of the transplant. In yet another embodiment, the tolerogenic or immunosuppressive cells can be administered to or generated in the recipient of the transplant after the administration of the transplant.

The present invention also includes the use of the tolerogenic or immunosuppressive cells as a therapy to inhibit graft versus host disease following transplantation. In this embodiment, the present invention encompasses a method of contacting a donor transplant with tolerogenic or immunosuppressive cells prior to transplantation of the transplant into a recipient. The tolerogenic or immunosuppressive cells serve to ameliorate, inhibit or reduce an adverse response by the donor transplant against the recipient. The tolerogenic or immunosuppressive cells can be obtained from any source, for example, from the transplant donor, the transplant recipient or an otherwise unrelated source (a different individual or species altogether) for the use of eliminating or reducing an unwanted immune response by a transplant against a recipient of the transplant. Accordingly, the tolerogenic or immunosuppressive cells can be autologous, allogeneic or xenogeneic to the transplant donor, the transplant recipient or an otherwise unrelated source. In an embodiment of the present invention, a transplant recipient suffering from graft versus host disease or at risk of suffering from graft versus host disease may be treated by administering the tolerogenic or immunosuppressive cells to the recipient or generating the tolerogenic or immunosuppressive cells in the recipient to reduce, inhibit or eliminate the severity of the graft versus host disease. In this embodiment of the invention, preferably, the tolerogenic or immunosuppressive cells may be obtained from the recipient prior to the transplantation and may be stored and/or expanded in culture to provide a reserve of tolerogenic or immunosuppressive cells in sufficient amounts for treating an ongoing graft versus host reaction. However, as discussed elsewhere herein, tolerogenic or immunosuppressive cells can be obtained from any source, for example, from the transplant donor, the transplant recipient or an otherwise unrelated source (a different individual or species altogether).

Based upon the disclosure herein, it is envisioned that the tolerogenic or immunosuppressive cells described herein can be used in conjunction with current modes of treatment, for example the use of immunosuppressive drug therapy for the treatment of host rejection to the donor tissue or graft versus host disease. An advantage of using tolerogenic or immunosuppressive cells in conjunction with immunosuppressive drugs in transplantation is that by using the tolerogenic or immunosuppressive cells to ameliorate the severity of the immune response in a transplant recipient, the amount of immunosuppressive drug therapy used and/or the frequency of administration of immunosuppressive drug therapy can be reduced. A benefit of reducing the use of immunosuppressive drug therapy is the alleviation of general immune suppression and unwanted side effects associated with immunosuppressive drug therapy.

The invention described herein encompasses, in particular, a method of preventing or treating transplant rejection and/or graft versus host disease by administering to a patient or generating in a patient tolerogenic or immunosuppressive cells described herein in a prophylactic or therapeutically effective amount for the prevention, treatment or amelioration of host rejection of the transplant and/or graft versus host disease. Based upon the present disclosure, a therapeutically effective amount of tolerogenic or immunosuppressive cells is an amount that inhibits or decreases the number of activated T cells, when compared with the number of activated T cells in the absence of the administration of tolerogenic or immunosuppressive cells. In the situation of host rejection of the transplant, an effective amount of tolerogenic or immunosuppressive cells is an amount that inhibits or decreases the number of activated T cells in the recipient of the transplant when compared with the number of activated T cells in the recipient prior to administration of the tolerogenic or immunosuppressive cells. In the case of graft versus host disease, an effective amount of tolerogenic or immunosuppressive cells is an amount that inhibits or decreases the number of activated T cells present in the transplant.

An effective amount of tolerogenic or immunosuppressive cells can be determined by comparing the number of activated T cells in a recipient or in a transplant prior to the administration of tolerogenic or immunosuppressive cells thereto, with the number of activated T cells present in the recipient or transplant following the administration of tolerogenic or immunosuppressive cells thereto. A decrease, or the absence of an increase, in the number of activated T cells in the recipient of the transplant or in the transplant itself that is associated with the administration of tolerogenic or immunosuppressive cells thereto, indicates that the number of tolerogenic or immunosuppressive cells administered is a therapeutic effective amount of tolerogenic or immunosuppressive cells.

Instead of administering tolerogenic or immunosuppressive cells to a patient, the invention may also be used to express CFLAR in cells of a mammal so as to generate tolerogenic or immunosuppressive cells in the mammal. This aspect of the invention relates to a kind of gene therapy in which therapeutic proteins are administered to a mammal by way of introducing a nucleic acid coding therefor into cells of a mammal. In all cases in which a nucleic acid is transfected into a cell, the nucleic acid sequence encoding CFLAR is operably linked to regulatory sequences required to achieve expression of the nucleic acid sequence in the cell. The nucleic acid construct is preferably provided as an expression vector that includes the coding sequence for CFLAR operably linked to essential regulatory sequences such that when the vector is transfected into the cell, the coding sequence will be expressed by the cell. The coding sequence is operably linked to the regulatory elements necessary for expression of that sequence in the cells. The nucleic acid that encodes CFLAR may be cDNA, genomic DNA, synthesized DNA or a hybrid thereof or an RNA molecule such as mRNA.

The nucleic acid construct includes the nucleotide sequence encoding CFLAR operably linked to the regulatory elements and may remain present in the cell as a functioning cytoplasmic molecule, a functioning episomal molecule or it may integrate into the cell's chromosomal DNA. Exogenous genetic material may be introduced into cells where it remains as separate genetic material in the form of a plasmid. Alternatively, linear DNA which can integrate into the chromosome may be introduced into the cell. When introducing DNA into the cell, reagents which promote DNA integration into chromosomes may be added. DNA sequences which are useful to promote integration may also be included in the DNA molecule. Alternatively, RNA may be introduced into the cell.

The basic approach of using tolerogenic or immunosuppressive cells of the invention to induce tolerance has widespread application as an adjunct to therapies which utilize a potentially immunogenic molecule for therapeutic purposes. For example, an increasing number of therapeutic approaches utilize a proteinaceous molecule, such as an antibody, fusion protein or the like, for treatment of a clinical disorder. A limitation to the use of such molecules therapeutically is that they can elicit an immune response directed against the therapeutic molecule in the subject being treated (e.g., the efficacy of Factor VIII in human subjects is hindered by the induction of an immune response against Factor VIII in the human subject). The present invention is an improvement on conventional protein therapy in the context of inducing tolerance against the administered molecule.

Administration of tolerogenic or immunosuppressive cells of the invention to inhibit T cell responses can be applied to these therapeutic situations to enable long term usage of the therapeutic molecule in the subject without elicitation of an immune response.

According to the invention, the term "disease" or "disorder" refers to any pathological state.

The term "inflammatory disease" refers to any disease, which is characterized by or associated with high levels of inflammation in tissues, in particular connective tissues, or degeneration of these tissues. A chronic inflammatory disease is a medical condition which is characterized by persistent inflammation. Examples of (chronic) inflammatory diseases include celiac disease, vasculitis, lupus, chronic obstructive pulmonary disease (COPD), irritable bowel disease, atherosclerosis, arthritis, ankylosing spondylitis, Crohn's disease, colitis, chronic active hepatitis, dermatitis and psoriasis.

The term "autoimmune disease" or "autoimmune disorder" refers to any disease/disorder in which the body produces an immunogenic (i.e. immune system) response to some constituent of its own tissue. In other words, the immune system loses its ability to recognize some tissue or system within the body as self and targets and attacks it as if it were foreign. Autoimmune diseases can be classified into those in which predominantly one organ is affected (e.g. hemolytic anemia and anti-immune thyroiditis), and those in which the autoimmune disease process is diffused through many tissues (e.g. systemic lupus erythematosus). For example, multiple sclerosis is thought to be caused by T cells attacking the sheaths that surround the nerve fibers of the brain and spinal cord. This results in loss of coordination, weakness, and blurred vision. Examples of autoimmune diseases include, but are not limited to, Addision's disease, alopecia areata, ankylosing spondylitis, autoimmune hepatitis, autoimmune parotitis, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, pemphigus vulgaris, psoriasis, rheumatic fever, rheumatoid arthritis, sarcoidosis, scleroderma, Sjogren's syndrome, spondyloarthropathies, thyroiditis, vasculitis, vitiligo, myxedema, pernicious anemia, ulcerative colitis, and type I diabetes mellitus, among others.

"Transplant" refers to a biocompatible lattice or a donor tissue, organ or cell, to be transplanted. An example of a transplant may include but is not limited to skin cells or tissue, bone marrow, and solid organs such as heart, pancreas, kidney, lung and liver. A transplant can also refer to any material that is to be administered to a host. For example, a transplant can refer to a nucleic acid or a protein.

The term "transplant rejection" refers to the rejection of a transplant such as a transplanted tissue or organ by the recipient's immune system, which may, ultimately, destroy the transplant.

The term "graft versus host disease" or "GvHD" as used herein is defined as a condition in a mammal including humans which occurs when allogeneic, immunologically active cells, such as those in bone marrow or spleen, are introduced, transplanted or grafted in a host resulting in a severe, even fatal, reaction due to immunoincompatibility between the host and graft wherein immunocompetent cells of the graft immunologically attack the host.

By "treat" is meant to administer an agent or composition as described herein to a subject in order to prevent or eliminate a disease, including arrest or slow a disease in a subject; inhibit or slow the development of a new disease in a subject; decrease the frequency or severity of symptoms and/or recurrences in a subject who currently has or who previously has had a disease; and/or prolong, i.e. increase the lifespan of the subject. In particular, the term "treatment of a disease" includes curing, shortening the duration, ameliorating, preventing, slowing down or inhibiting progression or worsening, or preventing or delaying the onset of a disease or the symptoms thereof.

By "being at risk" is meant a subject, i.e. a patient, that is identified as having a higher than normal chance of developing a disease compared to the general population. In addition, a subject who has had, or who currently has, a disease is a subject who has an increased risk for developing a disease, as such a subject may continue to develop a disease. Furthermore, a subject who has received or will receive a transplant is a subject who has an increased risk for developing GvHD.

In the context of the present invention, terms such as "protect", "prevent", "prophylactic", "preventive", or "protective" relate to the prevention or treatment or both of the occurrence and/or the propagation of a disease in a subject and, in particular, to minimizing the chance that a subject will develop a disease or to delaying the development of a disease. For example, a person at risk for a disease would be a candidate for therapy to prevent a disease.

A prophylactic administration of an agent or composition described herein, preferably protects the recipient from the development of a disease. A therapeutic administration of an agent or composition described herein, may lead to the inhibition of the progress/growth of the disease. This comprises the deceleration of the progress/growth of the disease, in particular a disruption of the progression of the disease, which preferably leads to elimination of the disease.

The agents and compositions provided herein may be used alone or in combination with conventional therapeutic regimens such as surgery, irradiation, chemotherapy and/or bone marrow transplantation (autologous, syngeneic, allogeneic or unrelated).

The term *"in vivo"* relates to the situation in a subject.

The terms "subject", "individual", "organism" or "patient" are used interchangeably and relate to vertebrates, preferably mammals. For example, mammals in the context of the present invention are humans, non-human primates, domesticated animals such as dogs, cats, sheep, cattle, goats, pigs, horses etc., laboratory animals such as mice, rats, rabbits, guinea pigs, etc. as well as animals in captivity such as animals of zoos. The term "animal" as used herein also includes humans. The term "subject" may also include a patient, i.e., an animal, preferably a human having a disease, preferably a disease as described herein.

The term "autologous" is used to describe anything that is derived from the same subject. For example, "autologous transplant" refers to a transplant of tissue or organs derived from the same subject. Such procedures are advantageous because they overcome the immunological barrier which otherwise results in rejection.

The term "allogeneic" is used to describe anything that is derived from different individuals of the same species. Two or more individuals are said to be allogeneic to one another when the genes at one or more loci are not identical.

The term "syngeneic" is used to describe anything that is derived from individuals or tissues having identical genotypes, i.e., identical twins or animals of the same inbred strain, or their tissues.

The term "heterologous" is used to describe something consisting of multiple different elements. As an example, the transfer of one individual's bone marrow into a different individual constitutes a heterologous transplant. A heterologous gene is a gene derived from a source other than the subject.

The agents and compositions described herein may be administered via any conventional route, including by injection or infusion. The administration may be carried out, for example, orally, intravenously, intraperitoneally, intramuscularly, subcutaneously or transdermally. In particular, the cells described herein can be administered systemically, i.e., parenterally, by intravenous injection or can be targeted to a particular tissue or organ, such as bone marrow. Furthermore, cells can be administered via a subcutaneous implantation of cells or by injection of the cells into connective tissue, for example, muscle.

The agents and compositions described herein are administered in effective amounts. An "effective amount" refers to the amount which achieves a desired reaction or a desired effect, e.g. a detectable level of immune suppression or tolerance, alone or together with further doses. In the case of treatment of a particular disease or of a particular condition, the desired reaction preferably relates to inhibition of the course of the disease. This comprises slowing down the progress of the disease and, in particular, interrupting or reversing the progress of the disease. The desired reaction in a treatment of a disease or of a condition may also be delay of the onset or a prevention of the onset of said disease or said condition.

An effective amount of an agent or composition described herein will depend on the condition to be treated, the severeness of the disease, the individual parameters of the patient, including age, physiological condition, size and weight, the duration of treatment, the type of an accompanying therapy (if present), the specific route of administration and similar factors.

Accordingly, the doses administered of the agents and compositions described herein may depend on various of such parameters. In the case that a reaction in a patient is insufficient with an initial dose, higher doses (or effectively higher doses achieved by a different, more localized route of administration) may be used.

The pharmaceutical compositions described herein are preferably sterile and contain an effective amount of the therapeutically active substance to generate the desired reaction or the desired effect.

The pharmaceutical compositions described herein are generally administered in pharmaceutically compatible amounts and in pharmaceutically compatible preparations. The term "pharmaceutically compatible" refers to a nontoxic material which does not interact with the action of the active component of the pharmaceutical composition. Preparations of this kind may usually contain salts, buffer substances, preservatives, carriers, and, where appropriate, other therapeutically active compounds. When used in medicine, the salts should be pharmaceutically compatible. However, salts which are not pharmaceutically compatible may used for preparing pharmaceutically compatible salts and are included in the invention. Pharmacologically and pharmaceutically compatible salts of this kind comprise in a nonlimiting way those prepared from the following acids: hydrochloric, hydrobromic, sulfuric, nitric, phosphoric, maleic, acetic, salicylic, citric, formic, malonic, succinic acids, and the like. Pharmaceutically compatible salts may also be prepared as alkali metal salts or alkaline earth metal salts, such as sodium salts, potassium salts or calcium salts.

The pharmaceutical compositions described herein may comprise a pharmaceutically compatible carrier. The term "carrier" refers to an organic or inorganic component, of a natural or synthetic nature, in which the active component is combined in order to facilitate application. According to the invention, the term "pharmaceutically compatible carrier" includes one or more compatible solid or liquid fillers, diluents or encapsulating substances, which are suitable for administration to a patient. The components of the pharmaceutical compositions described herein are usually such that no interaction occurs which substantially impairs the desired pharmaceutical efficacy.

The pharmaceutical compositions described herein may contain suitable buffer substances such as acetic acid in a salt, citric acid in a salt, boric acid in a salt and phosphoric acid in a salt.

The pharmaceutical compositions may, where appropriate, also contain suitable preservatives such as benzalkonium chloride, chlorobutanol, paraben and thimerosal.

The pharmaceutical compositions are usually provided in a uniform dosage form and may be prepared in a manner known per se. Pharmaceutical compositions described herein may be in the form of capsules, tablets, lozenges, solutions, suspensions, syrups, elixirs or in the form of an emulsion, for example.

Compositions suitable for parenteral administration usually comprise a sterile aqueous or nonaqueous preparation of the active compound, which is preferably isotonic to the blood of the recipient. Examples of compatible carriers and solvents are Ringer solution and isotonic sodium chloride solution. In addition, usually sterile, fixed oils are used as solution or suspension medium.

The present invention is further illustrated by the following examples which are not be construed as limiting the scope of the invention.

### EXAMPLES

The techniques and methods used herein are described herein or carried out in a manner known per se and as described, for example, in Sambrook et al., Molecular Cloning: A Laboratory Manual, 2nd Edition (1989) Cold Spring Harbor Laboratory Press, Cold Spring Harbor, N.Y. All methods including the use of kits and reagents are carried out according to the manufacturers' information unless specifically indicated.

### Example 1: The tranduction of human stem cells (CD34⁺ cells) and monocytes (CD14⁺ cells) with specific CFLAR_{L}- and CFLAR_{S}-encoding lentiviruses promotes an immunosuppressive phenotype.

We created two plasmid constructs containing the sequences of CFLAR_{L} (Figure 1A) or CFLARs (Figure 1B). The two constructs contained a reporter gene: a green fluorescent protein (GFP) with a tracking and marker function. For the production of lentiviral recombinant proteins, the method of precipitation with calcium chloride was used. Briefly, the plasmid vectors coding for key proteins for virus assembly were purchased by Takara company (Takara Clontech, CA, U.S.A.). Calcium chloride CaCl₂ (2 mM) was added to the solution containing the pGp vector, the pE-eco vector and the vector encoding CFLAR_{L} or CFLARs or luciferase (as control) (25 µg). Subsequently, the buffer HBS 2X (274 mM NaCl₂, 10 mM KCl, 1.5 mM Na₂HPO4, 12 mM dextrose, 42 mM Hepes, pH 7.1) was added under stirring. The tranfection mix was then added dropwise to the culture plate of 293T cells and the medium containing the viral particles was harvested after 48 hours, centrifuged and filtered in filters with 0.45µm pores to remove cells and debries and concentrated by ultracentrifugation at 50,000 g for 140 minutes at room temperature. The infection was carried out by spin-inoculation: cells (human stem cells or monocytes) were placed in a multi-well plate swinging bucket rotor and place in centrifuge; the plate was spinned at 2500rpm for 4 hours at 30°C. After this step, we set up immunosuppressive assays. In the case of human stem cells (Bone Marrow CD34⁺ cells, 1milion, cat. 2M-101C, Lonza, Switzerland), the infected CD34⁺ cells were *in vitro* differentiated in the presence of G-CSF (40 ng/ml; Miltenyi Biotec S.r.l., Italy) and GM-CSF (40 ng/ml, Miltenyi Biotec S.r.l., Italy) for four days to promote the acquisition of MDSC-related immunosuppressive activity. After four days, the cells were recovered and co-cultured in presence of activated cell trace-labeled PBMCs (Figure 2A, left panel). Briefly, PBMCs were recovered and washed in IMDM medium (Lonza, Switzerland), supplemented with 10% FBS (EuroClone, Italy), 100 U/ml penicillin/streptomycin (Lonza, Switzerland), β-mercaptoethanol (Sigma-Aldrich, Italy) and 10 mM HEPES (Lonza, Switzerland). PBMCs were resuspended at a final concentration of 10⁷ cells/ml in PBS and stained with 2.5 µM as final working concentration of Cell Trace Violet stock solution (Invitrogen Molecular Probe, U.K.), followed by 5 minutes incubation at 37°C, protected from light. The dye easily diffuses into cells, where it is cleaved by intracellular esterases to yield a highly fluorescent compound. This compound covalently binds to intracellular amines, resulting in stable, well-retained fluorescent staining. After every division, the dye distributes equally in daughter cells, so the cellular fluorescence decreases by half. In a flow cytometry histogram, discrete peaks toward the left-hand side appear and represent every cell division or generation. Cells were then washed and resuspended in culture medium. Labeled "target" PBMCs were stimulated with coated 1 µg/ml anti-CD3 (clone, OKT-3; eBioscience, CA, U.S.A.) and 5 µg/ml soluble anti-CD28 (clone, CD28.2, eBioscience, CA, U.S.A.) for four days and co-cultured with "effectors" *in vitro* differentiated CD34⁺ cells at different effectors to target ratios in 384 flat bottom well plates (BD Biosciences, NJ, U.S.A.). Cell cultures were incubated at 37°C and 5% CO₂ in arginine and glutamine-free-RPMI (Biochrom AG, Germany), supplemented with 2 mM L-glutamine, 150µM arginine, 10% FBS (Sigma-Aldrich, MO, U.S.A.), 10 U/ml penicillin and streptomycin (Lonza, Switzerland), and 0,1 mM HEPES (Lonza, Switzerland). At the end of the culture, cells were stained with PE-Cy7 conjugated anti-CD3 (UCHT1, eBioscience, CA, U.S.A.) and all samples were acquired with a FACS-CantoII (BD Biosciences, NJ, U.S.A.) and cell trace signal of gated lymphocytes was analyzed with FlowJo software (Treestar Inc.). The extent of cell proliferation was quantified analyzing the number of proliferating cells, assumed to be 100% without *in vitro* differentiated CD34⁺ cells. We demonstrated that that CFLAR up-regulation enhanced the immunosuppressive ability *in vitro* differentiate MDSC suggesting a novel biological role for this protein. In fact, CFLAR-expressing CD34⁺ cells displayed a significantly augmented suppressive activity compared to luciferase-expressing CD34⁺ cells (Figure 2A, right panel). To validate further these data, we infected immunomagnetically (cat. 130-050-201; Miltenyi Biotec S.r.l., Italy) purified CD14⁺ monocytes from buffy coats using, which do not usually show any immune suppressive functions, with either CFLAR- or luciferase-expressing lentiviruses and co-cultured them in presence of cell trace-labeled PBMCs activated by anti-human CD3/CD28 (Figure 2B, left panel). Also in this experimental setting, CFLAR-infected CD14⁺ cells acquired a stronger suppressive ability compared to luciferase-infected monocytes (Figure 2B, right panel). This powerful immunosuppressive function did not correlate with a better survival of CFLAR-expressing CD14⁺ cells compared to controls, since the percentage of CD14⁺7-AAD⁻ ANNEXIN-V⁻ cells after the *in vitro* co-culture was comparable (data not shown) using flow cytometry through APC-AnnexinV apoptosis Detection Kit with 7-AAD (cat. 640930, Biolegend, CA, U.S.A.). All samples were acquired with a FACS-CantoII (BD Biosciences, NJ, U.S.A.) and analyzed with FlowJo software (Treestar Inc.).

### Example 2: The enforced CFLAR expression on monocytes drives a MDSC-like molecular program that control the immunosuppressive function.

To identify better the CFLAR-activated molecular pathway underlying the immunosuppressive functional program in myeloid cells, we performed a transcriptome analysis of CFLAR- and luciferase-infected CD14⁺ cells isolated from four healthy donors. Briefly, total RNA was isolated using TRIzol reagent (Life technology, CA, U.S.A.) and RNA integrity assessed using Agilent-2100-Bioanalyzer (Agilent Technologies, CA, U.S.A.). The RNA was further purified with RNeasy MinElute Cleanup kit (Qiagen, Venlo, Netherlands) and cDNA was synthesized and amplified from total purified RNA with Ovation Pico WTA System V2 (NuGEN, CA, U.S.A.). All the samples were hybridized to Affymetrix U133 PLUS 2.0 arrays and scanned with an Affymetrix GCS 3000 7G scanner. Sample and genes are clustered using Pearson correlation coefficient and average as distance metric and linkage, respectively. As shown in Figure 3, the supervised clustering (q.val<0.05, fold change>2) generated a list of 750 up-regulated (Figure 4) and 1,130 down-regulated genes (data not shown) in CFLAR-infected monocytes compared to controls. In particular, these differentially expressed genes resulted significantly enriched in categories involved in inflammation pathways, Notch-related pathways and IL-10 related pathway. Moreover, the CFLAR enforced-expression activates the up-regulation of MDSC-related immunosuppressive genes such as SOCS2, FAS, CCR7, CCL5, NF-kB, STAT3, CD38, CD274, CD273, IL4R, IL6, IL10, CFS3, PTGS2 and IDOL Some of putative CFLAR-related targets were tested and validated (Figure 5). In particular we verified by Real Time PCR that CFLAR-transduced monocytes showed the up-regulation of both CFLAR_{L} and CFLARs, as well as the up-regulation of IDOL For this aim we isolated total RNA from both CFLAR- or luciferase-infected monocytes by TRIzol reagent (Life technology, CA, U.S.A.) and RNA integrity assessed using Agilent-2100-Bioanalyzer (Agilent Technologies, CA, U.S.A.). The RNA was further purified with RNeasy MinElute Cleanup kit (Qiagen, Venlo, Netherlands) and cDNA was synthesized and amplified from total purified RNA with Ovation Pico WTA System V2 (NuGEN, CA, U.S.A.). All the samples were used to set up a Real Time PCR with custom primers designed by ABI Biosystems by Primer Express. Post qRT-PCR analysis to quantify relative gene expression was performed by the comparative Ct method (2^{-ΔΔCt}). As shown in Figure 5A, the CFLAR-transduced monocytes expressed significantly high levels of putative target genes. Moreover we validated that CFLAR-transduced CD14⁺ cells secreted significantly higher level of IL-10 than luciferase-transduced CD14⁺ cells through ELISA (Enzyme Linked ImmunoSorbent Assay) assay (R&D System, MN, U.S.A.). In short, this assay consists in transferring the culture medium in a specific 96-well plate previously incubated with purified antibody specific for IL-10. In some wells, in place of the sample, the calibration curve was plated using 1:2 scalar dilution of the cytokine in question. After 2 hours of incubation at 37°C, the plate was marked with the biotinylated anti-IL-10 antibody for 1 hour, then followed by 30 minutes of incubation at room temperature with strepatavidin bound to peroxidise. The peroxidise reaction with its substrate (TMB-3,3',5,5'-tetramethylbenzidine) allows the quantification of the cytokine of interest by reading at 450 nm. Finally, we verified that the enforced CFLAR-expression on monocytes promoted the up-regulation of specific surface markers by flow cytometry. Briefly, CD14⁺ cells after 24 hours from infection, as previously described, were resuspended in 100 µl of FACS buffer (0.9% NaCl solution containing 2% BSA and 0.02% NaN₃; both from Sigma-Aldrich) with mouse anti-human CD273-PE (cat. 557926, BD Biosciences, NJ, U.S.A.), mouse anti-human CD274-APC (cat. 228489, BD Biosciences, NJ, U.S.A.), mouse anti-human CD80-APC (cat. 305220, Biolegend CA, U.S.A.), anti-human CD163-PE (cat. 556018, BD Biosciences, NJ, U.S.A.), anti-human CD44-PE (cat. 103024, Biolegend, NJ, U.S.A.), anti-human CD38-APC (cat. 554262, BD Biosciences, NJ, U.S.A.) and anti-human CD124-APC (cat. FAB23OP, R&D System, MN, U.S.A.). All samples were acquired with a FACS-CantoII (BD Biosciences, NJ, U.S.A.) and analyzed with FlowJo software (Treestar Inc.).

### Example 3: The adoptive transfer of CFLAR-expressing monocytes controls the progression of GvHD in immunodeficient mice transplanted with human PBMCs.

On the basis of these observations, we decided to assess *in vivo* the immune suppressive functions of CFLAR-infected CD14⁺ cells (as previously described) to control the development of graft versus host disease (GvHD) using a xenogeneic mouse model. For this purpose, we intravenously (i.v.) injected sub-lethally irradiated (using ¹³⁷Cs-source irradiator), immunodeficient NOD.Cg-*Prkdc*^{scid} Il2rg^{tmlSug}/JicTac (NOG) (purchased by Taconic, NY, U.S.A.) mice with 10⁶ human PBMCs isolated from buffy coats. When the frequency of circulating human CD3⁺ lymphocytes reached a percentage ~5% of total cells, we started treating the mice by i.v. transfer of 10⁶ CFLAR- or luciferase-expressing monocytes. Briefly by retro orbital bleeding we isolated 100µl of blood; red-cells were eliminated by ACK (Ammonium-Chloride-Potassium) Lysing Buffer and cells were stained with rat anti-mouse CD45-APC (clone A20, Biolegend, NJ, U.S.A.), anti-human CD45-PerPC-Cy5.5 (clone 2D1, Biolegend, NJ, U.S.A.) and anti-human CD3-PE-Cy7 (clone UHCT1, Biolegend, NJ, U.S.A.). All samples were acquired with a FACSCanto II (BD Biosciences, NJ, USA) and analyzed with FlowJo software (Treestar Inc.). The treatment was repeated four times, on day 21, 28, 42 and 49 after the initial challenge and the mice were monitored for the immunological, clinical and histopathological GvHD score (Figure 6A). The injection of CFLAR-infected CD14⁺ cells, efficiently reduced the GvHD development, resulting in an improved long-term survival of transplanted mice compared to GvHD-undergoing mice treated with luciferase-expressing monocytes or untreated (Figure 6B). In addition, the histologic analysis and the quantification of tissue-infiltrating human CD3⁺ T cells, revealed a lower aggressive GvHD pathology in all analyzed organs of mice treated with CFLAR-expressing monocytes (Figure 6C). Briefly, tissues were removed at necroscopy and fixed in 10% buffered formalin and embedded in paraffin. Histopathologic evaluation was performed on standard hematoxylin- and-eosin sections. Pathological score was evaluated by two pathologists blinded using the standard line guide previously published (Cooke KR, Kobzik L, Martin TR, Brewer J, Delmonte J, Crawford JM, Ferrara JLM: An experimental model of idiopathic pneumonia syndrome after bone marrow transplantation: The roles of minor H antigens and endotoxin. Blood 8:3230, 199). Finally, paraffin sections of spleen, liver, lung, skin, colon, kidney and stomach have been used for the immunohistochemistry: sections were stained with anti-human CD3 (clone ab5690, Abeam, United Kingdom) antibody to quantify human tissues-infiltrating lymphocytes.

To check the ability of CFLAR-expressing monocyes to control the *in vivo* expansion of T cells in GvHD-undergoing mice, we verified the presence of human CD3⁺ cell frequency in mice treated four times at day 21, 28, 42 and 49 from PBMCs engraftment (Figure 7A). After only two treatments, GvHD-undergoing mice treated with CFLAR-expressing monocytes showed a lower human T cells frequency compared to luciferase-treated ones (Figure 7B). In particular, GvHD-undergoing mice were sacrificed at day 35 after PBMC engraftment and we evaluated the human T cell frequency by flow cytometry in the blood (Figure 7B i), spleen (Figure 7B ii) and bone marrow (Figure 7B iii). For blood analysis, by retro orbital bleeding, we isolated 100µl of blood; red-cells were eliminated by ACK (Ammonium-Chloride-Potassium) Lysing Buffer and cells were stained with PE-Cy7 conjugated anti-CD3 (UCHT1, eBioscience, CA, U.S.A.) antibody. For spleen analysis, fresh splenocytes (10⁶/sample) were resuspended in 100 µl of FACS buffer with anti-human FcγR blocking solution (cat. 130-046-702, Miltenyi Biotec S.r.l., Italy) for 10 min at room temperature to reduce the nonspecific staining. After washing, cells were resuspended in FACS buffer and labeled with anti-human PE-Cy7 conjugated anti-CD3 (UCHT1, eBioscience, CA, U.S.A.) antibody. Finally for bone marrow cells analysis, tibias and femurs from GvHD-undergoing mice were removed using sterile techniques and BM was flushed. Red-cells were eliminated by ACK (Ammonium-Chloride-Potassium) Lysing Buffer and cells were stained with anti-human PE-Cy7 conjugated anti-CD3 (UCHT1, eBioscience, CA, U.S.A.) antibody. All samples were acquired with a FACSCanto II (BD Biosciences, NJ, USA) and analyzed with FlowJo software (Treestar Inc.). Moreover we evaluated also that activation state of human T cells. Briefly, splenocytes of treated GvHD-undergoing mice were stimulated or not (resting condition) *in vitro* for 24 hours with phorbol 12-myristate 13-acetate (PMA, 50 ng/ml) and ionomycin (1 µg/ml). Splenocytes were washed, stained with surface antibodies (anti-human Pacific Blue conjugated anti-CD8 (cat. 34417, Biolegend, CA, U.S.A.) antibody), fixed and permeabilized using Perm/Wash Kit (cat. 554723, BD Biosciences, NJ, USA) and incubated with PE-labeled hIFN-γ antibody (cat. 506506, Biolegend, CA, U.S.A.) (Figure 7B iiii). All samples were acquired with a FACSCanto II (BD Biosciences, NJ, USA) and analyzed with FlowJo software (Treestar Inc.). We demonstrated that T cells isolated from CFLAR-treated mice showed a statistically lower frequency of human CD8⁺IFNγ⁺ cells compared to luciferase-treated ones revealing a possible exhaustion or no-activated state mediated by CFLAR-monocytes. Finally we evaluated that the CFLAR-expressing monocyte injection in GvHD-undergoing mice induced the expansion of circulating T regulatory cell (Treg) expansion compared to luciferase-expressing monocytes (Figure 7C).

### Example 4: Rosa26.vFLIP;LysMcre mouse model

In order to understand better the CFLAR-induced molecular pathway, we took advantage of a transgenic mouse model expressing the viral form of CFLAR: the Rosa26.vFLIP knock-in mice. The Kaposi's sarcoma-associated herpesvirus encodes a FADD-like interferon converting enzyme or caspase 8 (FLICE) inhibitory protein (vFLIP) in its genome. The Rosa26.vFLIP knock-in mice were bred with mice expressing the Cre recombinase under the control of the endogenous *Lyz*2 promoter (LysM-Cre), thus resulting in mice with vFLIP expression restricted to the myeloid cell lineage. Bone marrow cells and splenocytes were obtained from both Rosa26.vFLIP;LysMcre and Rosa26.vFLIP mice and we isolated by fluorescence-activated cell sorting (FACS) the CD11B⁺ Ly6G⁻ Ly6C^{high} and the CD11B⁺ Ly6G⁺ Ly6C^{low} subsets. The immunosuppressive activity of these cells was then evaluated plating the freshly isolated myeloid cells in 96 wells plate at a final concentration of 24% of total cells in culture in presence of splenocytes from transgenic 37B7 mice (a mouse model that present all CD8⁺ T cell with an engineered T cell receptor (TCR) specific for the melanoma-associated antigen TRP2, labelled with CFSE 1 µM and diluted 1:10 with CD45.1⁺ splenocytes, in the presence of TRP-2₁₈₀₋₁₈₈ peptide (1 µg/ml). After 3 days of co-culture, we performed FACS analysis with a FACS-Canto (BD Biosciences, NJ, U.S.A.) and, to evaluate the suppression of proliferation, plots were gated on CD8⁺ T cells and the percentage of cells that had diluted CFSE was evaluated using FlowJo software (Treestar Inc.). The percentage of proliferating cells was then used to calculate the percent of suppression of proliferation using the following formula: 1 - (% proliferation with myeloid cells / % proliferation without myeloid cells) × 100 (Figure 8).

### Example 5: c-FLAR induces an immunosuppressive program independently of its anti-apoptotic functions.

Starting from healthy donor (HD) buffy coats, we purified and infected CD14⁺ monocytes with either c-FLAR- or luciferase-encoding lentiviruses and demonstrated that the immune regulatory property of c-FLAR did not correlate with a better survival of c-FLAR-expressing monocytes compared to controls, both when monocytes were cultured alone and when they were cultured with CD3/CD28-activated PBMCs. Indeed, the percentage of alive (7-AAD⁻ ANNEXIN-V⁻) CD14⁺ cells at different time points is not significantly different between c-FLAR- or luciferase-monocytes (Figure 9a), indicating that the immune regulatory activity can be separated from the anti-apoptotic properties of c-FLAR, at least in the case of mature monocytes. In addition, to discriminate between the pro-survival and the induction of immunosuppressive function, we generated lentiviral expressing-vectors of other anti-apoptotic genes (i.e. Bcl-2 and Bcl-xL) and tested the immunosuppressive properties after their enforced expression in human monocytes. As shown in Figure 9b, only the enforced expression of c-FLAR is significantly able to suppress *in vitro* T cell activation and proliferation.

### Example 6: Enforced c-FLAR expression in monocytes drives MDSC-associated molecular programs.

By using gene ontology (GO) enrichment analysis, the differentially expressed genes were significantly enriched in categories involved in inflammation, cytokine network and genes up-regulated in response to interferon proteins, as well as associated to the IL-10 pathway and, overall, to NP-κB-related pathway (Figure 10a,b).

### Example 7: c-FLAR activates the canonical NF-κB pathway in myeloid cells.

The enforced expression of c-FLAR triggers the canonical NF-κB pathway. Gene expression data suggested an unpredicted transcriptional and signaling activity of c-FLAR, possibly linked to the NF-κB pathway. To elucidate the molecular programs under c-FLAR control, we transiently transfected THP1 monocytic cells with *in vitro* transcribed (IVT) c-FLAR-encoding RNA. The rational was to induce a sustained and viral-independent production of the target protein; moreover, the IVT-RNA does not activate innate immunity receptors and type I interferon production. Enforced introduction of a synthetic c-FLAR RNA into THP1 cells triggered an immunosuppressive program consistent with the data shown thus far with lentivirus expression vectors (Figure 11a). Transfection of c-FLAR RNA significantly promoted the nuclear translocation of the NF-κB subunits p65 and p50, mediators of the canonical NF-κB activation pathway, while no difference in the p52 subunit translocation was detected (Figure 11b). To test whether NF-κB signalling was required for the immune regulatory program downstream of FLAR, we silenced the kinases IKKα (encoded by Chuk), and IKKβ (encoded by Ikbkb), both indispensable for canonical NF-κB activation. We demonstrated that the immunosuppressive activity of v-FLAR-expressing Ly6C⁺ cells isolated from Tg mice was significantly reduced after silencing either IKKα or IKKβ (Figure 11c). Collectively our data indicate that c-FLAR activates a signaling pathway that controls an immunosuppressive program in monocytes through NF-κB activation.

In particular, the present invention pertains to the following:
1. A population of cells comprising myeloid cells or progenitor cells thereof which are engineered so as to increase the cellular level of cellular FLICE [Fas-associated death domain (FADD)-like IL-1β-converting enzyme]-inhibitory protein (CFLAR).
2. The population of cells of item 1 wherein increasing the cellular level of CFLAR increases the immunosuppressive activity of the cells.
3. The population of cells of item 1 or 2 wherein the myeloid cells or progenitor cells thereof are engineered so as to increase cellular expression of CFLAR.
4. The population of cells of any one of items 1 to 3 wherein the myeloid cells or progenitor cells thereof are transfected with nucleic acid encoding CFLAR.
5. The population of cells of item 4 wherein said transfection is a stable or transient transfection.
6. The population of cells of item 4 or 5 wherein the myeloid cells or progenitor cells thereof are transfected using viral vectors as carrier such as lentiviral vectors.
7. The population of cells of item 4 or 5 wherein said nucleic acid is RNA.
8. The population of cells of any one of items 1 to 7 wherein the CFLAR is CFLAR_{L} and/or CFLARs.
9. The population of cells of any one of items 1 to 8 wherein the myeloid cells or progenitor cells thereof are isolated and/or purified cells, in particular isolated and/or purified by magnetic cell sorting.
10. The population of cells of any one of items 1 to 9 wherein the myeloid cells or progenitor cells thereof are monocytes.
11. The population of cells of any one of items 1 to 10 wherein the myeloid cells or progenitor cells thereof are CD14+ monocytes.
12. The population of cells of any one of items 1 to 11 wherein the myeloid cells or progenitor cells thereof are CD14+ monocytes isolated from buffy coats.
13. The population of cells of any one of items 1 to 9 wherein the myeloid cells or progenitor cells thereof are stem cells.
14. The population of cells of any one of items 1 to 9 and 13 wherein the myeloid cells or progenitor cells thereof are bone marrow CD34+ cells.
15. The population of cells of any one of items 1 to 9, 13 and 14 wherein the myeloid cells or progenitor cells thereof are bone marrow CD34+ cells isolated from bone marrow mononuclear cells (MNC).
16. The population of cells of any one of items 1 to 15 wherein the cells have been treated so as to promote the acquisition of immunosuppressive activity.
17. The population of cells of any one of items 1 to 16 wherein treatment so as to promote the acquisition of immunosuppressive activity comprises *in vitro* differentiation in the presence of G-CSF and GM-CSF.
18. The population of cells of any one of items 1 to 17 for therapy.
19. The population of cells of item 18 wherein the therapy requires immunosuppression.
20. The population of cells of any one of items 1 to 17 for treating a patient in need of immunosuppression.
21. The population of cells of item 20 which is autologous to the patient.
22. A pharmaceutical composition comprising the population of cells of any one of items 1 to 17.
23. The pharmaceutical composition of item 22 for therapy.
24. The pharmaceutical composition of item 23 wherein the therapy requires immunosuppression.
25. The pharmaceutical composition of item 22 for treating a patient in need of immunosuppression.
26. The pharmaceutical composition of item 25 wherein the population of cells is autologous to the patient.
27. Use of the population of cells of any one of items 1 to 17 for the production of a medicament for therapy.
28. The use of item 27 wherein the therapy requires immunosuppression.
29. Use of the population of cells of any one of items 1 to 17 for the production of a medicament for treating a patient in need of immunosuppression.
30. The use of item 29 wherein the population of cells is autologous to the patient.
31. A method for treating a patient in need of immunosuppression comprising administering the population of cells of any one of items 1 to 17.
32. The method of item 31 wherein the population of cells is autologous to the patient.
33. A method for treating a patient in need of immunosuppression comprising increasing the cellular level of CFLAR in myeloid cells or progenitor cells thereof of the patient.
34. The method of item 33 wherein increasing the cellular level of CFLAR increases the immunosuppressive activity of the cells.
35. The method of item 33 or 34 wherein increasing the cellular level of CFLAR in myeloid cells or progenitor cells thereof of the patient comprises administering a nucleic acid encoding CFLAR.
36. The method of item 35 wherein the nucleic acid encoding CFLAR transfects myeloid cells or progenitor cells thereof.
37. The method of item 36 wherein said transfection is a stable or transient transfection.
38. The method of item 37 wherein the myeloid cells or progenitor cells thereof are transfected using viral vectors as carrier such as lentiviral vectors.
39. The method of any one of items 35 to 37 wherein said nucleic acid is RNA.
40. The method of any one of items 33 to 39 wherein the CFLAR is CFLAR_{L} and/or CFLARs.
41. The method of any one of items 33 to 40 wherein the myeloid cells or progenitor cells thereof are monocytes.
42. The method of any one of items 33 to 41 wherein the myeloid cells or progenitor cells thereof are CD14+ monocytes.
43. The method of any one of items 33 to 40 wherein the myeloid cells or progenitor cells thereof are stem cells.
44. The method of any one of items 33 to 40 and 43 wherein the myeloid cells or progenitor cells thereof are bone marrow CD34+ cells.
45. A nucleic acid encoding CFLAR for therapy.
46. The nucleic acid of item 45 wherein the therapy requires immunosuppression.
47. A nucleic acid encoding CFLAR for treating a patient in need of immunosuppression.
48. A pharmaceutical composition comprising a nucleic acid encoding CFLAR.
49. The pharmaceutical composition of item 48 for therapy.
50. The pharmaceutical composition of item 49 wherein the therapy requires immunosuppression.
51. The pharmaceutical composition of item 48 for treating a patient in need of immunosuppression.
52. Use of a nucleic acid encoding CFLAR for the production of a medicament for therapy.
53. The use of item 52 wherein the therapy requires immunosuppression.
54. Use of a nucleic acid encoding CFLAR for the production of a medicament for treating a patient in need of immunosuppression.

## Claims

1. A population of cells for use in therapy, said population comprising myeloid cells or progenitor cells thereof which are engineered by *in vivo* transfection so as to increase the cellular level of cellular FLICE [Fas-associated death domain (FADD)-like IL-1β-converting enzyme]-inhibitory protein (CFLAR), wherein said population comprises myeloid cells or progenitor cells thereof comprising a recombinant nucleic acid encoding CFLAR, wherein the therapy comprises treating a patient in need of immunosuppression or wherein the therapy comprises treatment of a transplant recipient or of graft-versus-host disease or of an inflammatory disease or of an autoimmune disease, wherein the inflammatory disease is preferably celiac disease, vasculitis, lupus, chronic obstructive pulmonary disease (COPD), irritable bowel disease, atherosclerosis, arthritis, ankylosing spondylitis, Crohn's disease, colitis, chronic active hepatitis, dermatitis or psoriasis and wherein the autoimmune disease is preferably Addision's disease, alopecia areata, ankylosing spondylitis, autoimmune hepatitis, autoimmune parotitis, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, pemphigus vulgaris, psoriasis, rheumatic fever, rheumatoid arthritis, sarcoidosis, scleroderma, Sjogren's syndrome, spondyloarthropathies, thyroiditis, vasculitis, vitiligo, myxedema, pernicious anemia, ulcerative colitis, or type I diabetes mellitus.

2. The population of cells for the use of claim 1, wherein the myeloid cells or progenitor cells thereof have been transfected using a viral vector comprising said nucleic acid encoding CFLAR.

3. The population of cells for use of claim 1, wherein said nucleic acid is RNA.

4. The population of cells for use of any one of claims 1 to 3, wherein the CFLAR is CFLAR_{L} and/or CFLARs.

5. The population of cells for use of any one of claims 1 to 4, wherein the myeloid cells or progenitor cells thereof are isolated and/or purified cells, in particular isolated and/or purified by magnetic cell sorting, wherein the myeloid cells or progenitor cells thereof are preferably monocytes, more preferably CD14+ monocytes, or stem cells, or bone marrow CD34+ cells.

6. The population of cells for use of any one of claims 1 to 5, wherein said bone marrow CD34+ cells have been isolated from bone marrow mononuclear cells (MNC).

7. A pharmaceutical composition for use in therapy, said pharmaceutical composition comprising a population of cells comprising myeloid cells or progenitor cells thereof which are engineered by *in vitro* transfection so as to increase the cellular level of cellular FLICE [Fas-associated death domain (FADD)-like IL-β-converting enzyme]-inhibitory protein (CFLAR), wherein said population comprises myeloid cells or progenitor cells thereof comprising a recombinant nucleic acid encoding CFLAR wherein the therapy comprises treating a patient in need of immunosuppression or wherein the therapy comprises the treatment of a transplant recipient or of graft-versus-host disease or of an inflammatory disease or of an autoimmune disease, wherein the inflammatory disease is preferably celiac disease, vasculitis, lupus, chronic obstructive pulmonary disease (COPD), irritable bowel disease, atherosclerosis, arthritis, ankylosing spondylitis, Crohn's disease, colitis, chronic active hepatitis, dermatitis or psoriasis and wherein the autoimmune disease is preferably Addision's disease, alopecia areata, ankylosing spondylitis, autoimmune hepatitis, autoimmune parotitis, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, pemphigus vulgaris, psoriasis, rheumatic fever, rheumatoid arthritis, sarcoidosis, scleroderma, Sjogren's syndrome, spondyloarthropathies, thyroiditis, vasculitis, vitiligo, myxedema, pernicious anemia, ulcerative colitis, or type I diabetes mellitus.

8. The pharmaceutical composition for use of claim 7 or the population of cells for use of any one of claims 1 to 6, wherein the population of cells is autologous to the patient.

9. The pharmaceutical composition for use of claim 7 or 8, or the population of cells for use of any one of claims 1 to 6, comprising administering the pharmaceutical composition or the population of cells.

10. The pharmaceutical composition for use of any one of claims 7 to 9, wherein the cells have been treated so as to promote the acquisition of immunosuppressive activity, the treatment comprising *in vitro* differentiation in the presence of G-CSF and GM-CSF.

11. A transposon comprising a nucleic acid encoding CFLAR, or a pharmaceutical composition comprising said transposon, for use in therapy, wherein the therapy comprises treating a patient in need of immunosuppression or wherein the therapy comprises the treatment of a transplant recipient or of graft-versus-host disease or of an inflammatory disease or of an autoimmune disease, wherein the inflammatory disease is preferably celiac disease, vasculitis, lupus, chronic obstructive pulmonary disease (COPD), irritable bowel disease, atherosclerosis, arthritis, ankylosing spondylitis, Crohn's disease, colitis, chronic active hepatitis, dermatitis or psoriasis and wherein the autoimmune disease is preferably Addision's disease, alopecia areata, ankylosing spondylitis, autoimmune hepatitis, autoimmune parotitis, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, pemphigus vulgaris, psoriasis, rheumatic fever, rheumatoid arthritis, sarcoidosis, scleroderma, Sjogren's syndrome, spondyloarthropathies, thyroiditis, vasculitis, vitiligo, myxedema, pernicious anemia, ulcerative colitis, or type I diabetes mellitus.

12. The nucleic acid encoding CFLAR for use of claim 11, or the pharmaceutical composition for use of claim 11, wherein the therapy comprises administering the nucleic acid encoding CFLAR or the pharmaceutical composition to a patient and wherein the nucleic acid transfects myeloid cells or progenitor cells thereof in the patient.
